# EUROPÄISCHE PATENTSCHRIFT

(11) **EP 1 124 841 B1**
(45) Veröffentlichungstag und Bekanntmachung des Hinweises auf die Patenterteilung: **02.04.2003**
(21) Anmeldenummer: 99949001.4
(22) Anmeldetag: 15.10.1999
(51) Int. Cl.: C07J 41/00, A61K 31/575, C07J 9/00

(54) **SUBSTITUIERTE PHENYL-ALKENOYLGUANIDINE, VERFAHREN ZU IHRER HERSTELLUNG, IHRE VERWENDUNG ALS MEDIKAMENTE ODER DIAGNOSTIKA SOWIE SIE ENTHALTENDE MEDIKAMENTE**
SUBSTITUTED PHENYL ALKENOYL GUANIDINES, METHOD FOR THE PRODUCTION THEREOF, USE THEREOF AS MEDICAMENTS OR DIAGNOSTIC AGENTS AND MEDICAMENTS THAT CONTAIN THEM
PHENYL-ALCENOYLGUANIDINES SUBSTITUEES, LEURS PROCEDES DE PRODUCTION, LEUR UTILISATION COMME MEDICAMENTS OU AGENTS DE DIAGNOSTIC, ET MEDICAMENTS LES CONTENANT

(30) Priorität: 28.10.1998 DE 19849722
(43) Veröffentlichungstag der Anmeldung: 22.08.2001
(73) Patentinhaber: Aventis Pharma Deutschland GmbH, 65929 Frankfurt am Main (DE)
(72) Erfinder: WEICHERT, Andreas, D-63329 Egelsbach (DE); ENHSEN, Alfons, D-64572 Büttelborn (DE); FALK, Eugen, D-60529 Frankfurt (DE); JANSEN, Hans-Willi, D-65527 Niedernhausen (DE); KRAMER, Werner, D-55130 Mainz-Laubenheim (DE); SCHWARK, Jan-Robert, D-65779 Kelkheim (DE); LANG, Hans, Jochen, D-65719 Hofheim (DE)
(86) Internationale Anmeldenummer: EP9907828
(87) Internationale Veröffentlichungsnummer: WO00024761

(56) Entgegenhaltungen:
- EP-A- 0 624 594

## Beschreibung

Die Erfindung betrifft substituierte Phenyl-alkenoylguanidine und deren pharmazeutisch verträgliche Salze und physiologisch funktionelle Derivate.

Die Bildung von Gallensteinen wird neben einer Reihe von Faktoren wesentlich durch die Zusammensetzung der Galle bestimmt, im besonderen durch die Konzentration und das Verhältnis von Cholesterin, Phospholipiden und Gallensalzen. Voraussetzung für die Bildung von Cholesteringallensteinen ist das Vorhandensein einer an Cholesterin übersattigten Galle (Lit. Carey, M. C. and Small, D.M. (1978) The physical chemistry of cholesterol solubility in bile. Relationship to gallstone formation and dissolution in man, J. Clin. Invest. 61: 998-1026).

Gallensteine werden bislang vorwiegend chirurgisch entfernt, so daß ein großer therapeutischer Bedarf zur medikamentösen Gallensteinauflösung und zur Prävention der Gallensteinbildung besteht.

Der Erfindung lag die Aufgabe zugrunde, Verbindungen zur Verfügung zu stellen, die in der Lage sind, die Bildung von Gallensteinen zu verhindern, indem sie die Übersättigung der Galle mit Cholesterin verhindern, oder indem sie die Bildung von Cholesterinkristallen aus übersättigten Gallen verzögern.

Die Erfindung betrifft daher Verbindungen der Formel I 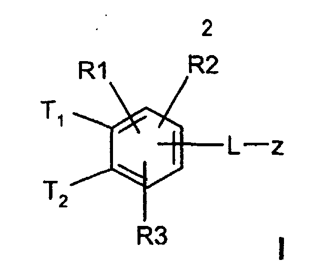 worin bedeuten
- T1 und T2: unabhängig voneinander 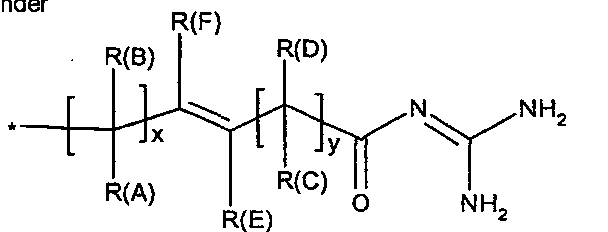 oder Wasserstoff, wobei T1 und T2 nicht gleichzeitig Wasserstoff sein können; 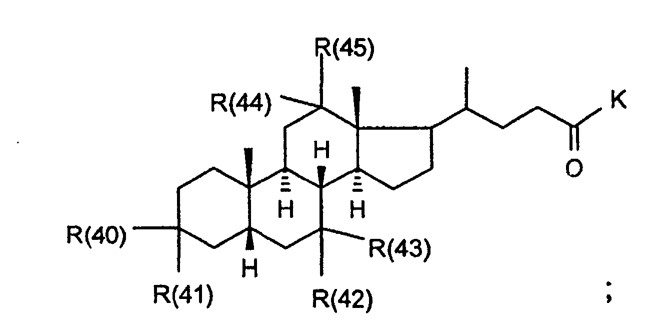
- R(A), R(B), R(C), R(D): unabhängig voneinander Wasserstoff, F, Cl, Br, I, CN, OH, NH₂, -(C₁-C₈)-Alkyl, -O-(C₁-C₈)-Alkyl, wobei die Alkylreste ein oder mehrfach mit F substituiert sein können, (C₃-C₈)-Cycloalkyl, Phenyl, Benzyl, NHR(7), NR(7)R(8), O-(C₃-C₆)-Alkenyl, O-(C₃-C₈)-Cycloalkyl, O-Phenyl, O-Benzyl, wobei der Phenylkern bis zu dreifach substituiert sein kann mit F, Cl, CF₃, Methyl, Methoxy, NR(9)R(10);
- R(7), R(8): unabhängig voneinander Wasserstoff, -(C₁-C₈)-Alkyl, wobei der Alkylrest ein oder mehrfach mit F substituiert sein kann, (C₃-C₈)-Cycloalkyl, (C₃-C₆)-Alkenyl, (C₃-C₈)-Cycloalkyl, Phenyl, Benzyl, wobei der Phenylkern bis zu dreifach substituiert sein kann mit F, Cl. CF₃, Methyl, Methoxy, NR(9)R(10); oder
- R(7), R(8): bilden gemeinsam eine Kette von 4 oder 5 Methylengruppen, von denen eine CH₂-Gruppe durch Sauerstoff, Schwefel, NH, N-CH₃ oder N-Benzyl ersetzt sein kann;
- R(9), R(10): unabhängig voneinander Wasserstoff, (C₁-C₄)-Alkyl, (C₁-C₄)-Perfluoralkyl;
- x: Null, 1 oder 2;
- y: Null, 1 oder 2;
- R(E), R(F): unabhängig voneinander Wasserstoff, F, Cl, Br, I, CN, (C₁-C₈)-Alkyl, O-(C₁-C₈)-Alkyl, wobei der Alkylrest ein oder mehrfach mit F substituiert sein kann,
(C₃-C₈)-Cycloalkyl, O-(C₃-C₆)-Alkenyl, O-(C₃-C₈)-Cycloalkyl, O-Phenyl, O-Benzyl, wobei der Phenylkem bis zu dreifach substituiert sein kann mit F, Cl, CF₃, Methyl, Methoxy, NR(9)R(10);
- R(1), R(2), R(3): unabhängig voneinander Wasserstoff, F, Cl, Br, I, CN, -(C₁-C₈)-Alkyl, -O-(C₁-C₈)-Alkyl, wobei die Alkylreste ein oder mehrfach mit F substituiert sein können,
-(C=O)-N=C(NH₂)₂,-(SO₀₋₂)-(C₁-C₈)-Alkyl,-(SO₂)-NR(7)R(8), -O-(C₀-C₈)-Alkylen-phenyl, -(C₀-C₈)-Alkylen-phenyl, wobei die Phenylkerne bis zu 3-fach substituiert sein können mit F, Cl, CF₃, Methyl, Methoxy, -(C₀-C₈)-Alkylen-NR(9)R(10);
- L: -O-, -NR(47)-, -(C₁-C₈)-Alkylen-, -(C₂-C₈)-Alkenylen-, -(C₂-C₈)-Alkinylen-, -COO-, -CO-NR(47)-, -SO₂-NR(47)-, -O-(CH₂)ₙ-O-, -NR(47)-(CH₂)ₙ-O-, -NR(48)-CO-(CH₂)ₙ-O-, -CO-N R(48)-(CH₂)ₙ-O-, -O-CO-(CH₂)ₙ-O-, -SO₂-NR(48)-(CH₂)ₙ-O-, -NR(48)-CO-CH₂-CH₂-CO-NR(48)-(CH₂)ₙ-O-, -NR(48)-CO-CH=CH-CO-NR(48)-(CH₂)ₙ-O-, -NR(48)-SO₂-(CH₂)ₙ-O-
- R(47): Wasserstoff, (C₁-C₈)-Alkyl, R(48)-CO-, Phenyl, Benzyl;
- R(48): Wasserstoff, (C₁-C₈)-Alkyl, Phenyl und Benzyl, wobei der Phenylkem bis zu 3-fach substituiert sein kann mit F, Cl, CF₃,Methyl, Methoxy;
- n: 1 bis 8;
- R(40) bis R(45): unabhängig voneinander Wasserstoff, -OR(50), -SR(50), NHR(50), -NR(50)₂, -O-(CO)-R(50), -S-(CO)-R(50), -NH-(CO)-R(50),-O-PO-(OR(50))-OR(50), -O-(SO₂)-OR(50), -R(50), eine Bindung zu L; oder
- R(40) und R(41),: R(42) und R(43), R(44) und R(45) bilden jeweils gemeinsam den Sauerstoff einer Carbonylgruppe;
wobei immer genau einer der Reste R(40) bis R(45) die Bedeutung einer Bindung zu L hat;
- K: -OR(50), -NHR(50), -NR(50)₂, -HN-CH₂-CH₂-CO₂H, -HN-CH₂-CH₂-SO₃H, -NH-CH₂-COOH, -N(CH₃)CH₂CO₂H, -HN-CH(R46)CO₂H, -OKa, wobei Ka ein Kation bedeutet, wie z.B. ein Alkali- oder Erdalkaliion oder ein quartäres Ammoniumion;
- R(46): Wasserstoff, C₁-C₄-Alkyl, Benzyl, -CH₂-OH, H₃CSCH₂CH₂-, HO₂CCH₂-, HO₂CCH₂CH₂-;
- R(50): Wasserstoff, (C₁-C₄)-Alkyl, Phenyl oder Benzyl, wobei der Phenylkem bis zu 3-fach substituiert sein kann mit F, Cl, CF₃,Methyl, Methoxy;
sowie deren pharmazeutisch verträgliche Salze und physiologisch funktionelle Derivate.

Bevorzugt sind Verbindungen der Formel I 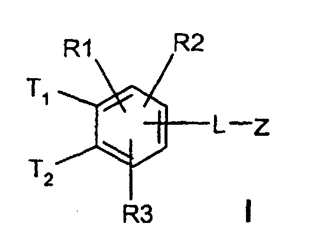 worin bedeuten
- T1 und T2: unabhängig voneinander gleich 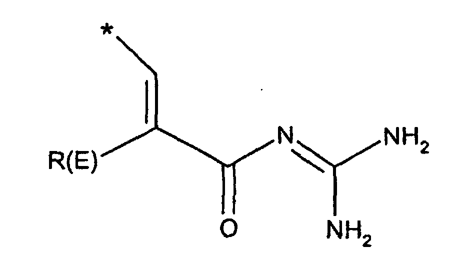 oder Wasserstoff, wobeiT1 und T2 nicht gleichzeitig Wasserstoff sein können;
- L-z: 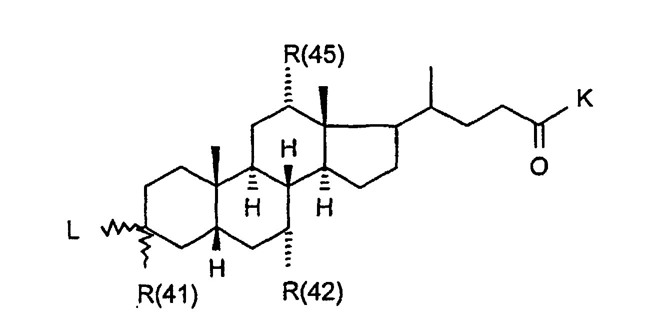
- R(E): Wasserstoff, F, Cl, CN, (C₁-C₄)-Alkyl, , -O-(C₁-C₄)-Alkyl, wobei die Alkylreste ein oder mehrfach mit F substituiert sein können, (C₃-C₆)-Cycloalkyl, O-(C₃-C₈)-Alkenyl, O-(C₃-C₆)-Cycloalkyl, O-Phenyl, O-Benzyl, wobei der Phenylkem bis zu dreifach substituiert sein kann mit F, Cl, CF₃, Methyl, Methoxy, NR(9)R(10);
- R(9), R(10): unabhängig voneinander Wasserstoff, CH₃, CF₃;
- R(1), R(2), R(3): unabhängig voneinander Wasserstoff, F, Cl, CN, -SO₂-(C₁-C₄)-Alkyl, -SO₂-N((C₁-C₄)-Alkyl)₂, -SO₂-NH(C₁-C₄)-Alkyl, -SO₂-NH₂, -SO₂-(C₁-C₄)-Alkyl, -(C₁-C₄)-Alkyl, -O-(C₁-C₄)-Alkyl, wobei die Alkylreste ein oder mehrfach mit F substituiert sein können,
-O-(C₀-C₄)-Alkylen-phenyl, -(C₀-C₄)-Alkylen-phenyl, wobei die Phenylkerne bis zu 3-fach substituiert sein können mit F, Cl, CF₃, Methyl, Methoxy;
- L: -O-, -NR(47)-, -(C₁-C₄)-Alkylen-, -(C₂-C₄)-Alkenylen-, -(C₂-C₄)-Alkinylen-, -COO-, -CO-NR(47)-, -SO₂-NR(47)-, -O-(CH₂)ₙ-O-, -NR(47)-(CH₂)ₙ-O-, -NR(48)-CO-(CH₂)ₙ-O-, -CO-NR(48)-(CH₂)ₙ-O-, -SO₂-NR(48)-(CH₂)ₙ-O-;
- R(47): Wasserstoff, (C₁-C₄)-Alkyl, R(48)-CO-, Phenyl, Benzyl;
- R(48): Wasserstoff, (C₁-C₄)-Alkyl, Phenyl und Benzyl, wobei der Phenylkern bis zu 3-fach substituiert sein kann mit F, Cl, CF₃, Methyl, Methoxy;
- n: 1-4;
- R(41), R(42), R(45): unabhängig voneinander Wasserstoff, -OR(50), NHR(50), -NR(50)₂, -O-(CO)-R(50), -NH-(CO)-R(50);
- R(50): Wasserstoff, (C₁-C₄)-Alkyl, Phenyl oder Benzyl,wobei der Phenylkem bis zu 3-fach substituiert sein kann mit F, Cl, CF₃, Methyl, Methoxy;
- K: -OR(50), -NHR(50), -NR(50)₂, -HN-CH₂-CH₂-CO₂H, -HN-CH₂-CH₂-SO₃H, -NH-CH₂-COOH, -N(CH₃)CH₂CO₂H, -OKa, wobei Ka ein Kation bedeutet, wie z.B. ein Alkali- oder Erdalkaliion oder ein quartäres Ammoniumion;
sowie deren pharmazeutisch verträgliche Salze und physiologisch funktionelle Derivate.

Besonders bevorzugt sind Verbindungen der Formel I 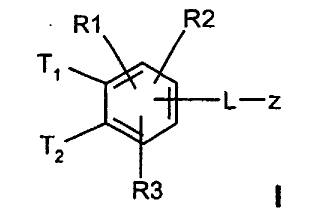 worin bedeuten
T1 und T2 unabhängig voneinander 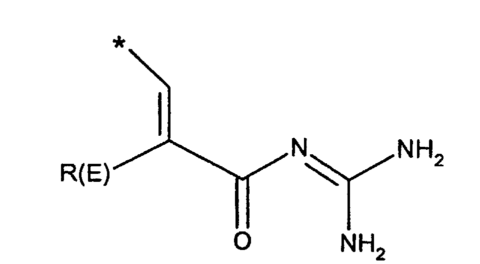 oder Wasserstoff, wobei T1 und T2 nicht gleichzeitig Wasserstoff sein können,
und L-z 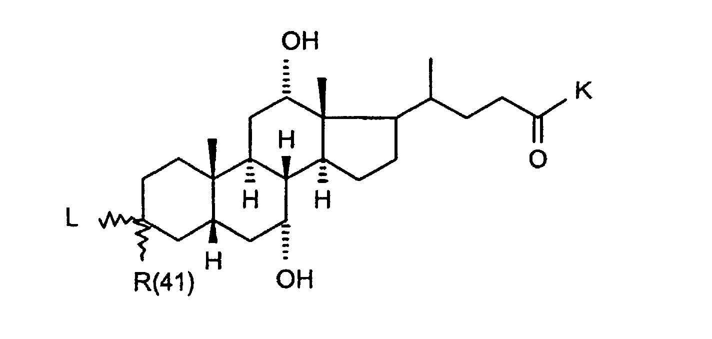
- R(E): Wasserstoff, F, Cl, CN, (C₁-C₄)-Alkyl, -O(C₁-C₄)-Alkyl, CF₃,-OCF₃;
- R(1), R(2): unabhängig voneinander Wasserstoff, F, Cl, CN, -SO₂-CH₃, SO₂NH₂-, -(C₁-C₄)-Alkyl, -O-(C₁-C₄)-Alkyl, wobei die Alkylreste ein oder mehrfach mit F substituiert sein können, -O-(C₀-C₄)-Alkylen-phenyl, -(C₀-C₄)-Alkylen-phenyl, wobei die Phenylkeme bis zu 3-fach substituiert sein können mit F, Cl, CF₃, Methyl, Methoxy;
- R(3): Wasserstoff,
- L: -O-, -NR(47)-, -CH₂-CH₂-, CH=CH-, -(C≡C)-, -COO-, -CO-NR(47)-, -SO₂-NR(47)-, -O-(CH₂)ₙ-O-, -NR(47)-(CH₂)ₙ-O-, -NR(48)-CO-(CH₂)ₙ-O-, -CO-NR(48)-(CH₂)ₙ-O-, -SO₂-NR(48)-(CH₂)ₙ-O-;
- R(47): Wasserstoff, (C₁-C₄)-Alkyl, R(48)-CO-, Phenyl, Benzyl;
- R(48): Wasserstoff, (C₁-C₄)-Alkyl, Phenyl und Benzyl, wobei der Phenylkem bis zu 3-fach substituiert sein kann mit F, Cl, CF₃, Methyl, Methoxy;
- n: 1-4;
- R(41): Wasserstoff, -OH;
- K: -OR(50), -NHR(50), -NR(50)₂, -HN-CH₂-CH₂-CO₂H, -HN-CH₂-CH₂-SO₃H, -NH-CH₂-COOH, -N(CH₃)CH₂CO₂H, -OKa, wobei Ka ein Kation bedeutet, wie z.B. ein Alkali- oder Erdalkaliion oder ein quartäres Ammoniumion;
- R(50): Wasserstoff, (C₁-C₄)-Alkyl, Phenyl oder Benzyl,wobei der Phenylkem bis zu 3-fach substituiert sein kann mit F, Cl, CF₃, Methyl, Methoxy;
sowie deren pharmazeutisch verträgliche Salze.

Ganz besonders bevorzugt sind Verbindungen der Formel I mit der Struktur Ia 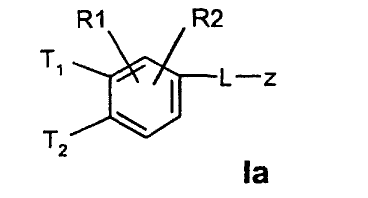 worin bedeuten
- T1 und T2: unabhängig voneinander 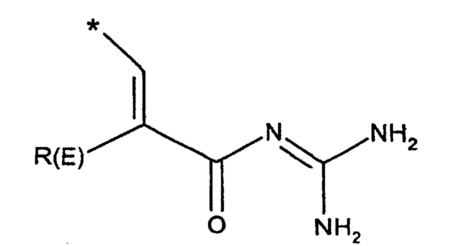 oder Wasserstoff, wobei T1 und T2 nicht gleichzeitig Wasserstoff sein können;
- L-z: 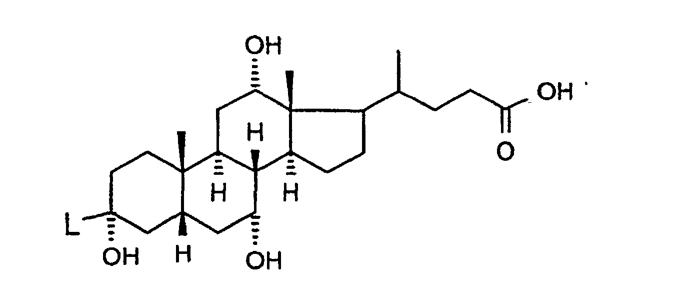
- L: ―C≡C―, -NH-CH₂-CH₂-O-;
- R(E): Wasserstoff, (C₁-C₄)-Alkyl;
- R(1), R(2): unabhängig voneinander Wasserstoff, F, Cl, CN, -SO₂CH₃, -(C₁-C₄)-Alkyl, -O-(C₁-C₄)-Alkyl, wobei die Alkylreste ein oder mehrfach mit F substituiert sein können;
sowie deren pharmazeutisch verträgliche Salze.

"*" markiert in in den obigen Formeln den Anknüpfungspunkt von T1 oder T2 an den Phenylring der Formel I.

Enthalten die Verbindungen der Formel I ein oder mehrere Asymmetriezentren, so können diese sowohl S als auch R konfiguriert sein. Die Verbindungen können als optische Isomere, als Diastereomere, als Racemate oder als Gemische derselben vorliegen.

Die Doppelbindungsgeometrie der Verbindungen der Formel I kann sowohl E als auch Z sein. Die Verbindungen können als Doppelbindungsisomere im Gemisch vorliegen.

Der Ausdruck " wobei der Alkylrest ein oder mehrfach mit F substituiert sein kann" umfasst auch perfluorierte Alkylreste.

Die bezeichneten Alkylreste können sowohl geradkettig wie verzweigt vorliegen.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches

Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-,Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Apfel-, Methansulfon-, Bernstein-, p-Toluolsulfon-, Wein- und Trifluoressigsäure. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natriumund Kaliumsalze) und Erdalkalisalze (wie Magnesium- und Calciumsalze).

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, Solvate und physiologisch funktionellen Derivate wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten.

Im allgemeinen liegt die Tagesdosis im Bereich von 0,1 mg bis 100 mg (typischerweise von 0,1 mg bis 50 mg) pro Tag pro Kilogramm Körpergewicht, z.B. 0,1-10 mg/kg/Tag. Tabletten oder Kapseln, können beispielsweise von 0,01 bis 100 mg, typischerweise von 0,02 bis 50 mg enthalten. Im Falle pharmazeutisch verträglicher Salze beziehen sich die vorgenannten Gewichtsangaben auf das Gewicht des vom Salz abgeleiteten Aminopropanol-lons. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale und perorale (z.B. sublinguale) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säureund magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Die Erfindung betrifft weiterhin ein Verfahren zur Herstellung einer Verbindung der Formel 1, dadurch gekennzeichnet, daß man eine Verbindung der Formel II, 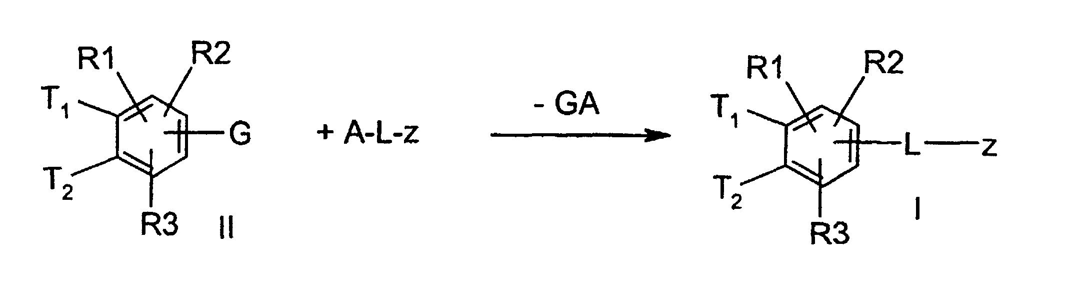 wobei T1, T2, R(1), R(2) und R(3) die oben angegebene Bedeutung haben und G für eine durch L-z austauschbare Funktionalität steht, in einer dem Fachmann bekannten Art und Weise mit einer Verbindung A-L-z zur Reaktion bringt, wobei GA abgespalten wird und eine Verbindung der Formel I entsteht.

Die Funktionalität G der Verbindung mit der Formel II kann beispielsweise die Bedeutung von Brom oder lod besitzen. Durch Pd(0)-Katalyse kann dann in bekannter Art und Weise die gewünschte C-C-Bindungsknüpfung erzielt werden.

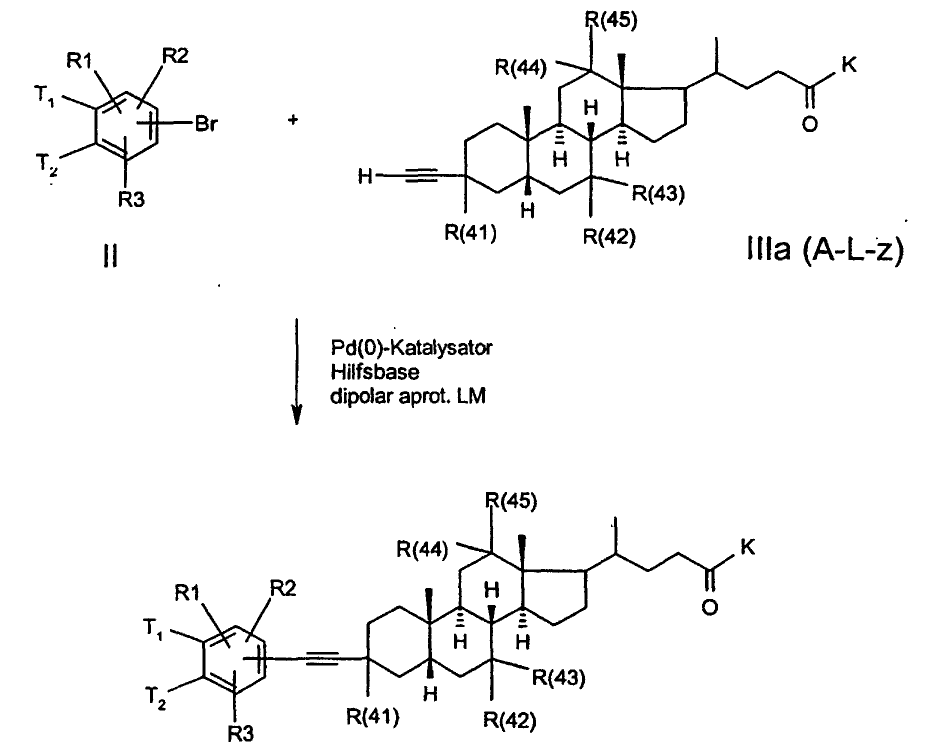

Die Acetylen-Gallensäurederivate der Formel III werden aus geeigneten Gallensäureketonen hergestellt. Dazu wird Lithiumacetylid analog zu bekannten Verfahren (US 5,641,767) an Ketogallensäuren addiert.

Die Verbindungen der Formel I und deren pharmazeutisch verträgliche Salze und physiologisch funktionelle Derivate zeichnen sich durch eine günstige Beeinflussung der Gallezusammensetzung und verhindern die Bildung von Gallensteinen, indem sie die Übersättigung der Galle mit Cholesterin verhindern, oder indem sie die Bildung von Cholesterinkristallen aus übersättigten Gallen verzögern. Die Verbindungen können allein oder in Kombination mit lipidsenkenden Wirkstoffen eingesetzt werden. Die Verbindungen eignen sich insbesondere zur Prophylaxe sowie zur Behandlung von Gallensteinen.

Die erfindungsgemäßen Verbindungen der Formel (I) gelangen in das hepatobiliäre System und wirken daher in diesen Geweben. So wird die Wasserabsorption aus der Gallenblase durch Inhibition des apikalen NHE-Antiports vom Subtyp 3 des Gallenblasenepithels gehemmt, was eine verdünnte Gallenflüssigkeit zur Folge hat.

Die biologische Prüfung der erfindungsgemäßen Verbindungen erfolgte durch Ermittlung der Inhibition des Natrium / Protonen-Austauscher Subtyp 3.

### 1. Testbeschreibung

Zur Bestimmung der IC₅₀-Werte für die Hemmung von humanem NHE-3 Protein (exprimiert in LAP1-Zellinie) wurde die Erholung des intrazellulären pHs (pHᵢ) nach einer Ansäuerung ermittelt, die bei funktionsfähigem NHE auch unter bicarbonatfreien Bedingungen einsetzt. Dazu wurde der pHᵢ mit dem pH-sensitiven Fluoreszenzfarbstoff BCECF (Calbiochem, eingesetzt wird die Vorstufe BCECF-AM) bestimmt. Die Zellen wurden zunächst mit BCECF beladen. Die BCECF-Fluoreszenz wurde in einem "Ratio Fluorescence Spectrometer" (Photon Technology International, South Brunswick, N.J., USA) bei Anregungswellenlängen von 505 und 440 nm und einer Emissionswellenlänge von 535 nm bestimmt und mittels Kalibrierungskurven in den pHᵢ umgerechnet. Die Zellen wurden bereits bei der BCECF-Beladung in NH₄Cl-Puffer (pH 7,4) inkubiert (NH₄Cl-Puffer: 115 mM NaCl, 20 mM NH₄Cl, 5 mM KCI, 1 mM CaCl₂, 1 mM MgSO₄, 20 mM Hepes, 5 mM Glucose, 1 mg/ml BSA; mit 1 M NaOH wird ein pH von 7,4 eingestellt). Die intrazelluläre Ansäuerung wurde durch Zugabe von 975 µl eines NH₄Cl-freien Puffers zu 25 µl Aliquots der in NH₄Cl-Puffer inkubierten Zellen induziert. Die nachfolgende Geschwindigkeit der pH-Erholung wurde 3 Minuten registriert. Für die Berechnung der inhibitorischen Potenz der getesteten Substanzen wurden die Zellen zunächst in Puffern untersucht, bei denen eine vollständige bzw. überhaupt keine pH-Erholung stattfand. Zur vollständigen pH-Erholung (100%) wurden die Zellen in Ma⁺-haltigem Puffer inkubiert (133,8 mM NaCl, 4,7 mM KCI, 1,25 mM CaCl₂, 1,25 mM MgCl₂, 0,97 mM Na₂HPO₄, 0,23 mM NaH₂PO₄, 5 mM Hepes, 5 mM Glucose, mit 1 M NaOH wird ein pH von 7,0 eingestellt). Für die Bestimmung des 0%-Wertes wurden die Zellen in einem Na⁺-freien Puffer inkubiert (133,8 mM Cholinchlorid, 4,7 mM KCI, 1,25 mM CaCl₂, 1,25 mM MgCl₂, 0,97 mM K₂HPO₄, 0,23 mM KH₂PO₄, 5 mM Hepes, 5 mM Glucose, mit 1 M NaOH wird ein pH von 7,0 eingestellt). Die zu testenden Substanzen wurden in dem Na⁺-haltigem Puffer angesetzt. Die Erholung des intrazellulären pHs bei jeder getesteten Konzentration einer Substanz wurde in Prozent der maximalen Erholung ausgedrückt. Aus den Prozentwerten der pH-Erholung wurde mittels des Programms SigmaPlot (Version 3.0, Jandel Scientific, USA) der IC₅₀-Wert der jeweiligen Substanz berechnet.

### Ergebnisse:

### Beispiel 1: IC₅₀ =1.7µM/I

Die nachfolgenden Beispiele dienen zur näheren Erläuterung der Erfindung, ohne dieselbe auf in den Beispielen beschriebene Produkte und Ausführungsformen einzuschränken.

### Liste der Abkürzungen:

| | |
|---|---|
| MeOH | Methanol |
| LAH | Lithiumaluminiumhydrid |
| DMF | N,N-Dimethylformamid |
| El | electron impact |
| Cl | Chemical lonisation |
| RT | Raumtemperatur |
| EE | Ethylacetat (EtOAc) |
| mp | Schmelzpunkt |
| HEP | n-Heptan |
| DME | Dimethoxyethan |
| ES | Elektronenspray |
| FAB | Fast Atom Bombardment |
| CH₂Cl₂ | Dichlormethan |
| THF | Tetrahydrofuran |
| eq. | Äquivalent |

Allgemeines Verfahren zur Kopplung von Arylhalogeniden und substituierten, terminalen Acetylenen:

Das Arylhalogenid (1 eq) wird zusammen mit einer Hilfsbase (4 eq) wie z.B. Triethylamin und einem Pd-Katalysator wie z.B. Palladium-bis-triphenylphosphinodichlorid (3 mol%) in DMF vorgelegt. Innerhalb von 0.5-3h wird das Acetylenderivat langsam zugegeben und falls nötig nochmals obige Menge an Katalysator zugesetzt. Die Reaktionstemperatur kann dabei RT überschreiten und annähernd 100°C erreichen, typischerweise liegt sie bei 60°C. Durch Zusatz von Essigester läßt sich das Rohprodukt präzipitieren und filtrieren. Eine sich anschließende Salzbildung wird durch Säurezugabe in Aceton erreicht.

### Beispiel 1:

4-{3β-[3,4-Bis-(3-guanidino-2-methyl-3-oxo-propenyl)-phenylethynyl]-3α,7α,12α-trihydroxy-10β,13β-dimethyl-hexadecahydro-cyclopenta[a]phenanthren-17-yl}pentansäure-diacetat, gelblicher Feststoff, Smp. 250°C (Zers.), MS: M⁺+H (FAB)=880.

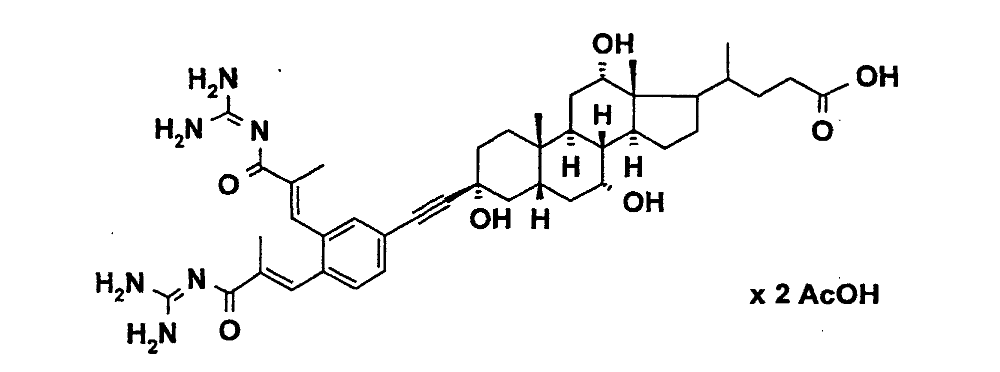

### Darstellung der Zwischenprodukte 1 und 2:

Zwischenprodukt 1: 3β-Acetylen-cholsäure

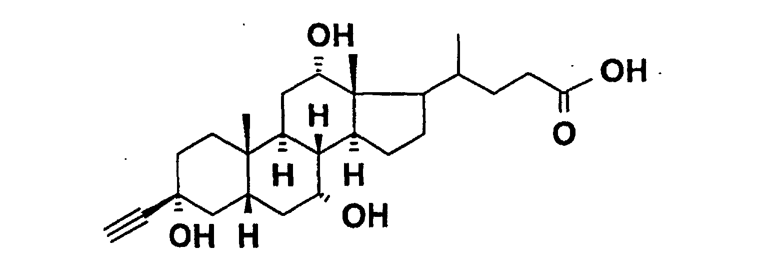

### Syntheseweg:

a) 3,7,12-Triacetylcholsäuremethylester
   90 g Cholsäuremethylester und 3.0 g Dimethylaminopyridin wurden in 500 ml Pyridin gelöst, mit 500 ml Acetanhydrid versetzt und über Nacht bei Zimmertemperatur gerührt. Es wurde auf Eiswasser gegossen und mit Ethylacetat (3x) extrahiert. Trocknen (MgSO₄) und Eindampfen der organischen Phase ergaben 92 g 3,7,12-Triacetylcholsäuremethylester, MS: M⁺+Li (FAB)=555.
b) 7,12-Diacetyl-cholsäuremethylester
   Bei 5°C wurden 150 ml Acetanhydrid langsam in 1.5 I Methanol zugetropft. Nach 15 Minuten wurden 92 g 3,7,12-Triacetylcholsäuremethylester zugegeben und 1 h bei Zimmertemperatur gerührt. Es wurde auf Eiswasser gegossen und mit Ethylacetat (3x) extrahiert. Die organische Phase wurde mit 1N Na₂CO₃-Lösung gewaschen, mit MgSO₄ getrocknet und eingedampft. Es wurden 85 g Rohprodukt erhalten, MS: M⁺+Li (FAB)=513.
c) 3-Keto-7,12-diacetyl-cholsäuremethylester
   85 g (168 mmol) 7,12-Diacetylcholsäuremethylester, 183.7 g Pyridiniumchlorochromat und 175 g Molekularsieb wurden in 2.5 I Dichlormethan 2 h bei Zimmertemperatur gerührt. Es wurde auf 7 I Diethylether gegossen, die Feststoffe abfiltriert. Das Lösungsmittel wurde eingedampft und der Rückstand in Ethylacetat gelöst. Nach Chromatographie über eine Florisil-Säule wurden 59.6 g Produkt erhalten, MS: M⁺+Li (FAB)=511.
d) 3β-Acetylen-7,12-diacetyl-cholsäuremethylester
   In 750 ml abs. Tetrahydrofuran wurde bei -55°C unter Argon 25 min Acetylen eingeleitet. Zu dieser Lösung wurden 145 ml 15% n-Butyllithium in Hexan zugetropft und 10 min nachgerührt. Anschließend wurden 45 g (89 mmol) 3-Keto-7,12-diacetylcholsäuremethylester zugegeben und 1.5 h bei -40°C gerührt. Zur Aufarbeitung wurden 500 ml gesättigte wäßrige Ammoniumchloridlösung zugegeben und mit Ethylacetat (3x) extrahiert, die organische Phase über MgSO₄ getrocknet und eingedampft. Der Rückstand wurde über Kieselgel chromatographiert (n-Heptan/ Ethylacetat 1:1). Es wurden 35.3 g Produkt erhalten, MS: M⁺+Li (FAB)=537.
e) 3β-Acetylen-cholsäure
   35.2 g (66 mmol) des Produkts aus d) wurden in 1 I Methanol gelöst, mit 300 ml 2N Natriumhydroxidlösung versetzt und 25 h unter Rückfluß erhitzt. Das Lösungsmittel wurde eingedampft, der Rückstand in Wasser gelöst und mit 2N Salzsäure bis pH 2 angesäuert. Der Niederschlag wurde abfiltriert und mit Wasser neutral gewaschen. Trocknung des Rückstandes ergab 14.6 g Produkt, MS: M⁺+Li (FAB)=439.

Zwischenprodukt 2: 1,2-Bis-[3-(E-2-methyl-propensäureguanidid)]-4-brom-benzoldihydrochlorid

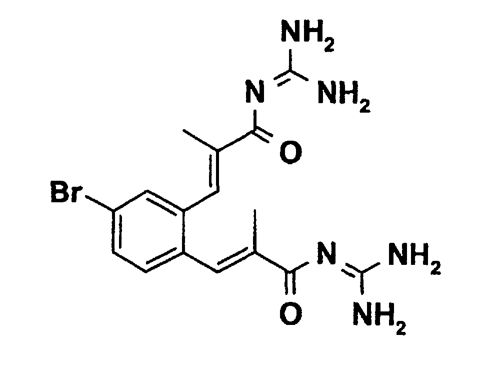

### Syntheseweg:

a) 4-Brom-1,2-phthaldialkohol aus 4-Brom-phthalsäuredimethylester gemäß Standardmethoden (z.B. Reduktion mit LAH), farbloses Öl; MS (Cl): M⁺+H=217.
b) 4-Brom-1,2-phthaldialdehyd aus 2a) durch z.B. Swem Oxidation unter Standardbedingungen, amorpher Feststoff, MS (Cl): M⁺+H=213.
c) 4-Brom-1,2-di-[3-(E-2-methyl-propensäureethylester)]-benzol durch Deprotonierung von 1 eq 2-Phosphonopropionsäuretriethylester mit 1 eq. n-Butyllithium in Hexan bei 0°C und anschließender Reaktion bei RT mit 0.5 eq. 4-Brom-1,2-phthaldialdehyd 2b). Nach vollständiger Abreaktion des Dialdehyds wurde mit Wasser aufgearbeitet und dreimal mit Toluol ausgeschüttelt. Nach Trocknen der vereinigten organischen Phasen über Magnesiumsulfat wurde das Lösungsmittel im Vakuum entfernt und das verbleibende Rohprodukt chromatographisch an Kieselgel mit EE/HEP-Gemischen als Eluent getrennt, farbloses Öl; MS (Cl): M⁺+H=381.
d) 4-Brom-1,2-di-[3-(E-2-methyl-propensäure)]-benzol aus 2c) durch Verseifung gemäß einer Standardmethode (Natriumhydroxid in Methanol), farbloser amorpher Feststoff, MS (ES): M⁺+H=325.
e) 1,2-Bis-[3-(E-2-methyl-propensäureguanidid)]-4-brom-benzol-dihydrochlorid aus 2d) gemäß allg. Variante, farbloser Feststoff; mp 240°C; MS (FAB): M⁺+H=407.
f) 4-{3β-[3,4-Bis-(3-guanidino-2-methyl-3-oxo-propenyl)-phenylethynyl]-3α,7α,12α-trihydroxy-10β,13β-dimethyl-hexadecahydro-cyclopenta[a]phenanthren-17-yl}pentansäure-diacetat aus 2e) und 3β-Acetylencholsäure mittels Pd(0)-Kopplung nach allg. Verfahren in DMF bei 60°C innerhalb 2h.

### Beispiel 2:

4-{3β-[3,4-Bis-(3-guanidino-2-methyl-3-oxo-propenyl)-phenylethynyl]-3α,7α,12α-trihydroxy-10β,13β-dimethyl-hexadecahydro-cyclopenta[a]phenanthren-17-yl}pentansäure-benzylester, gelblicher Feststoff, Smp. 155°C, MS: M⁺+H (ES)=849.

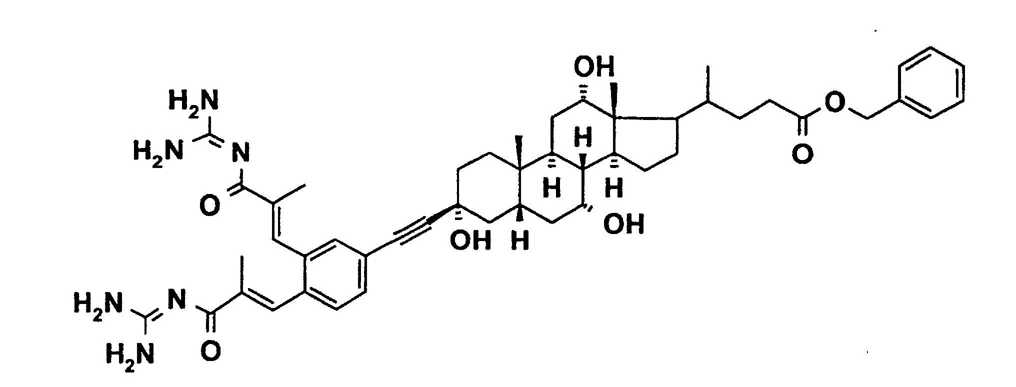

Synthese. analog Beispiel 1 unter Verwendung von 3β-Acetylencholsäurebenzylester.

### Beispiel 3:

4-{3β-[4-(3-Guanidino-3-oxo-propenyl)-phenylethynyl]-3α,7α,12α-trihydroxy-10β,13β-dimethyl-hexadecahydro-cyclopenta[a]phenanthren-17-yl}-pentansäurebenzylester, 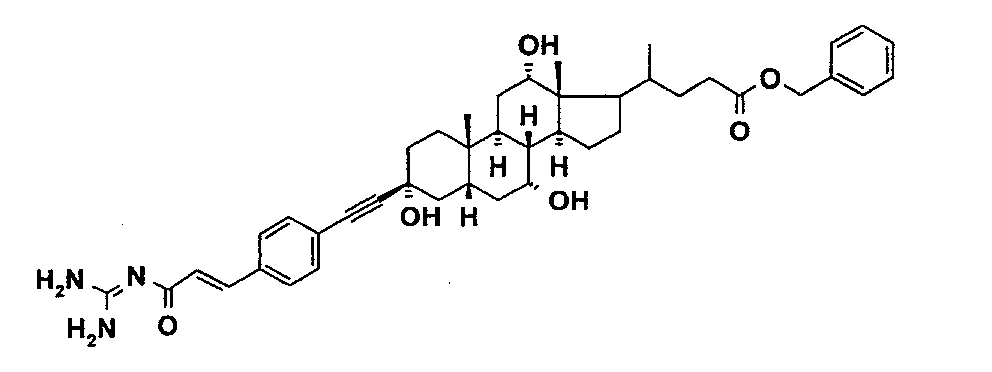 gelblicher Feststoff, Smp. 189°C, MS: M⁺+H (FAB)=710.

Synthese analog allg. Verfahren unter Verwendung von 4-Brom-zimtsäureguanidid und 3β-Acetylen-cholsäurebenzylester.

### Beispiel 4:

4-{3β-[4-(3-Guanidino-3-oxo-propenyl)-phenylethynyl]-3α,7α,12α-trihydroxy-10β, 13β-dimethyl-hexadecahydro-cyclopenta[a]phenanthren-17-yl}-pentansäuremethylester, gelblicher Feststoff, Smp. 60°C, MS: M⁺+H (FAB)=718.

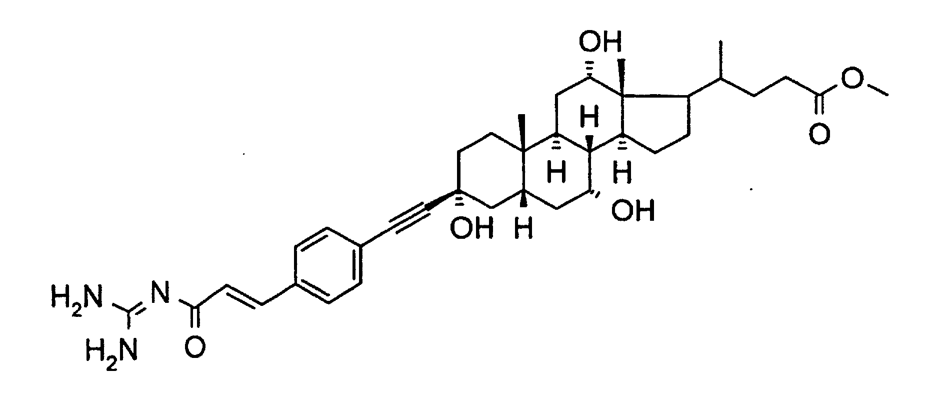

Synthese analog allg. Verfahren durch Reaktion von 4-Brom-zimtsäureguanidid und 3β-Acetylen-cholsäurebenzylester.

### Beispiel 5:

(4-{3β-[3,4-Bis-(3-guanidino-2-methyl-3-oxo-propenyl)-phenylethynyl]-3α,7α,12α-trihydroxy-10β, 13β-dimethyl-hexadecahydro-cyclopenta[a]phenanthren-17-yl}pentanoylamino)-essigsäure. 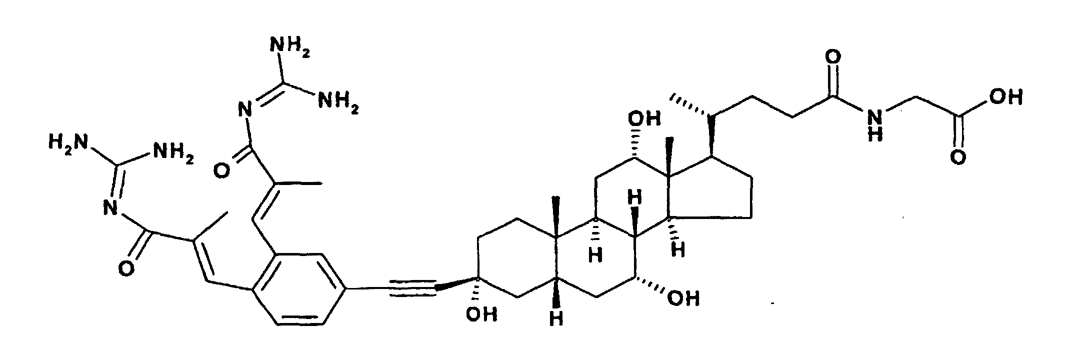
a) [4-(3β-Ethynyl-3α,7α,12α-trihydroxy-10β,13β-dimethyl-hexadecahydrocyclopenta[a]phenanthren-17-yl)-pentanoylamino]-essigsäure-methylester
   530 mg 3β-Acetylen-cholsäure (Zwischenprodukt 1e) sowie 510 µl Triethylamin werden in 30 ml THF gelöst und bei 0°C 175 µl Chlorameisensäureethylester zugetropft. 15 Minuten wird bei 0°C nachgerührt, anschließend eine Lösung von 340 mg Glycinmethylester-Hydrochlorid in 10 ml DMF zugetropft und 4 h bei RT gerührt. Mit 200 ml EE wird verdünnt und 2 mal mit je 50 ml einer 5% wäßrigen NaHSO₄-Lösung gewaschen. Über MgSO₄ wird getrocknet und das Solvens im Vakuum entfernt. Der Rückstand wurde in 100 ml EE ausgenommen und 3 mal mit je 50 ml einer gesättigten wäßrigen Na₂CO₃-Lösung gewaschen. Über MgSO₄ wird getrocknet und das Solvens im Vakuum entfernt. Chromatographie an Kieselgel mit EE/MeOH 10:1 und anschließend ein zweites Mal mit EE liefert 280 mg eines farblosen Schaumes.
   R_{f}(EE) = 0.37 MS (FAB): 518 (M+H)⁺
b) [4-(3β-Ethynyl-3α,7α,12α-trihydroxy-10β,13β-dimethyl-hexadecahydrocyclopenta[a]phenanthren-17-yl)-pentanoylamino]-essigsäure
   270 mg [4-(3β-Ethynyl-3α,7α,12α-trihydroxy-10β,13β-dimethyl-hexadecahydrocyclopenta[a]phenanthren-17-yl)-pentanoylamino]-essigsäure-methylester und 630 µl einer 1n wäßrigen NaOH-Lösung werden in 5 ml Ethanol gelöst und 16 h bei RT stehen gelassen. Das Solvens wird im Vakuum entfernt, der Rückstand mit 50 ml einer gesättigten wäßrigen NaH₂PO₄-Lösung aufgenommen und 3 mal mit je 50 ml EE extrahiert. Über MgSO₄ wird getrocknet und das Solvens im Vakuum entfernt. Man erhält 230 mg eines amorphen Feststoffs.
   R_{f}(Aceton/Wasser 10:1) = 0.25 MS (FAB): 502 (M+2Li)⁺
c) (4-{3β-[3,4-Bis-(3-guanidino-2-methyl-3-oxo-propenyl)-phenylethynyl]-3α,7α,12α-trihydroxy- 10β,13β-dimethyl-hexadecahydro-cyclopenta[a]phenanthren-17-yl}-pentanoylamino)-essigsäure
   230 mg [4-(3β-Ethynyl-3α,7α,12α-trihydroxy-10β,13β-dimethyl-hexadecahydrocyclopenta[a]phenanthren- 17-yl)-pentanoylamino]-essigsäure sowie 183 mg N-{3-[4-Brom-2-(3-guanidino-2-methyl-3-oxo-propenyl)-phenyl]-2-methyl-acryloyl}guanidin werden mittels Pd(0)-Kopplung nach allg. Verfahren in DMF bei 60°C innerhalb 3 h umgesetzt. Nach präparativer HPLC über C18 LiChrosorb mit Acetonitril/Wasser 2:4 + 0.1% Essigsäure + 0.1% Ammoniumacetat erhält man 70 mg eines amorphen Feststoffs.
   R_{f}(n-Butanol/Eisessig/Wasser 3:1:1) = 0.33 MS (ES): 816 (M+H)⁺

### Beispiel 6:

4-{3-[2-Fluor-4-(3-guanidino-2-methyl-3-oxo-propenyl)-phenylethinyl]-3,7,12-trihydroxy- 10,13-dimethyl-hexadecahydro-cyclopenta[a]phenanthren-17-yl}pentansäure 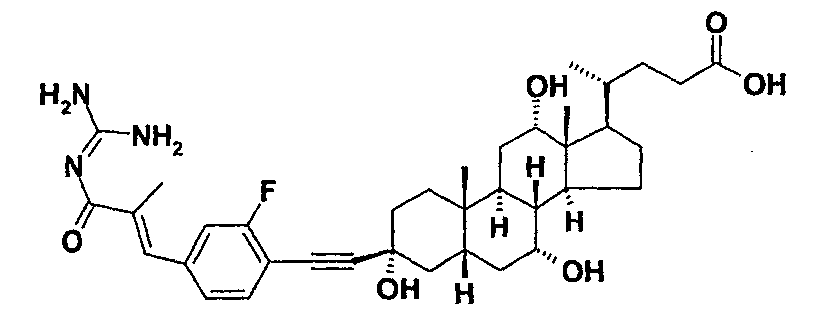
a) 3-(4-Brom-3-fluor-phenyl)-2-methyl-acrylsäure-butylester
   2 g 1-Brom-2-fluor-4-iod-benzol und 1,1 ml Diisopropylethylamin werden in 20 ml Dimethylacetamid (wasserfrei) gelöst und 5 Minuten lang ein leichter Argonstrom durch die Lösung geleitet. Anschließend werden 1,4 ml Acrylsäurebutylester und 10 mg 2,6-Di-*t*-butyl-4-methylphenol zugegeben und auf 100°C erwärmt. Schließlich werden weitere 4 ml Dimethylacetamid mittels eines Argonstroms entgast und 80 mg Trans-bis(□-acetato)bis[o-(di-o-tolylphosphino)benzyl]dipalladium (Tetrahedron Lett. 1996, 37(36), 6535-6538) darin suspendiert. Diese Suspension wird zur Mischung der übrigen Reaktionspartner addiert und 90 Minuten bei 140°C gerührt. Das Gemisch wird anschleißend mit 200 ml EE verdünnt, 2 mal mit je 100 ml Wasser und 1 mal mit 100 ml einer gesättigten wäßrigen NaCI-Lösung gewaschen. Über MgSO₄ wird getrocknet und das Solvens im Vakuum entfernt. Chromatographie an Kieselgel liefert 230 mg eines farblosen Öls.
   R_{f}(EE/HEP) = 0.27 MS (DCI): 315 (M+H)⁺
b) 3-{4-[17-(3-Carboxy-1-methyl-propyl)-3,7,12-trihydroxy-10,13-dimethylhexadecahydro- cyclopenta[a]phenanthren-3-ylethinyl]-3-fluor-phenyl}-2-methyl-acrylsäure-butylester
   64 mg Bis-(triphenylphosphin)-palladium(II)-chlorid, 17 mg Cul, 0,5 ml Triethylamin sowie 230 mg 3-(4-Brom-3-fluor-phenyl)-2-methyl-acrylsäure-butylester werden in 10 ml wasserfreiem DMF gelöst und bei 60°C innerhalb einer Stunde eine Lösung von 395 mg 3□-Acetylen-cholsäure in 10 ml wasserfreiem DMF zugetropft. Eine Stunde wird bei 60°C gerührt und dann erneut eine Lösung von 395 mg 3□-Acetylencholsäure in 10 ml wasserfreiem DMF bei 60 °C langsam zugetropft. Weitere 2 Stunden wird bei 60°C gerührt, anschließend nochmals 64 mg Bis-(triphenylphosphin)-palladium(II)-chlorid und 17 mg Cul zugegeben und erneut 2 Stunden bei 60°C gerührt. Schließlich werden weitere 80 mg 3□-Acetylen-cholsäure und 2 Stunden bei 60°C gerührt. Das Solvens wird im Vakuum entfernt, der Rückstand in 100 ml einer 5% wäßrigen NaHSO₄-Lösung aufgenommen und 3 mal mit je 100 ml EE extrahiert. Über Na₂SO₄ wird getrocknet und das Solvens im Vakuum entfernt. Chromatographie an Kieselgel mit EE/MeOH 5:1 liefert 90 mg einer wachsähnlichen Substanz.
   R_{f}(EE/MeOH 5:1) = 0.56 MS (FAB): 667 (M+H)⁺
c) 4-{3-[2-Fluor-4-(3-guanidino-2-methyl-3-oxo-propenyl)-phenylethinyl]-3,7,12-trihydroxy-10,13-dimethyl-hexadecahydrocyclopenta[a]phenanthren-17-yl}-pentansäure
   73 mg Guanidin-Hydrochlorid und 71 mg Kalium-*t*-butylat werden in 2 ml wasserfreiem DMF gelöst und 30 Minuten bei RT gerührt. Diese Suspension wird zu 85 mg 3-{4-[17-(3-Carboxy-1-methyl-propyl)-3,7,12-trihydroxy-10,13-dimethylhexadecahydro- cyclopenta[a]phenanthren-3-ylethinyl]-3-fluor-phenyl}-2-methylacrylsäure-butylester gespritzt und 5 Stunden bei 100°C gerührt. Nach Abkühlung werden 10 ml Wasser zugegeben, mit wäßriger HCI-Lösung auf pH=4 gestellt und 3 mal mit je 10 ml EE extrahiert. Über MgSO₄ wird getrocknet und das Solvens im Vakuum entfernt. Chromatographie an Kieselgel mit Aceton/Wasser 10:1 liefert 15,5 mg eines amorphen Feststoffs.
   R_{f}(Aceton/Wasser 10:1) = 0.19 MS (ES): 652 (M+H)⁺

### Beispiel 7:

4-(3-{2-[2,6-Difluoro-4-(3-guanidino-2-methyl-3-oxo-propenyl)-phenylamino]-ethoxy}-7,12-dihydroxy-10,13-dimethyl-hexadecahydro-cyclopenta[a]phenanthren-17-yl)-pentansäure 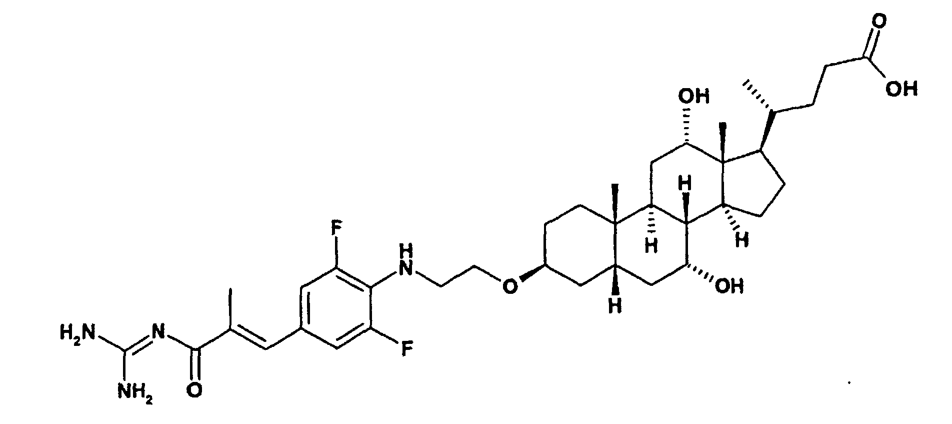
a) 4-(7,12-Dihydroxy-3-methanesulfonyloxy-10,13-dimethyl-hexadecahydrocyclopenta[a]phenanthren-17-yl)-pentansäure
   100 g Cholsäure werden in 500 ml Pyridin gelöst und bei 0°C 23,1 ml Mesylchlorid über einen Zeitraum von 30 Minuten zugetropft. 3 Stunden lang wird bei RT gerührt, anschließend bei 0°C auf eine Lösung von 400 ml H₂SO₄ in 3 I Wasser gegossen und 4 mal mit je 750 ml EE extrahiert. Über Na₂SO₄ wird getrocknet und das Solvens im Vakuum entfernt. Der Rückstand wird mit Diisopropylether zur Kristallisation gebracht und man erhält 117,1 g; mp 121°C (unter Zersetzung). R_{f}(EE/HEP/Essigsäure 5:5:1) = 0.31 MS (FAB): 487 (M+H)⁺
b) 4-[7,12-Dihydroxy-3-(2-hydroxy-ethoxy)-10,13-dimethyl-hexadecahydrocyclopenta[a]phenanthren-17-yl]-pentansäure-methylester
   116 g 4-(7,12-Dihydroxy-3-methanesulfonyloxy-10,13-dimethyl-hexadecahydrocyclopenta[a]phenanthren-17-yl)-pentansäure sowie 130 ml Triethylamin werden in 650 ml Glycol gelöst und 3 Stunden bei 100°C sowie 7,5 Stunden bei 115°C gerührt. Das Reaktionsgemisch wird bei 0°C auf eine Lösung von 400 ml H₂SO₄ in 3 I Wasser gegossen und 7 mal mit je 750 ml EE extrahiert. Über Na₂SO₄ wird getrocknet und das Solvens im Vakuum entfernt. Man erhält das Zwischenprodukt ZWP.
   Bei 0°C werden 130 ml Acetylchlorid zu 900 ml Methanol getropft. Dann wird eine Lösung von ZWP in 400 ml zugegeben und 6 Stunden bei RT gerührt. 60 Stunden wird bei RT stehen gelassen, dann auf 2.6 I Wasser gegossen und 8 mal mit je 500 ml Diisopropylether (DIP) extrahiert. Die organische Phase wird anschließend noch 6 mal mit je 600 ml einer halbgesättigten wäßrigen einer NaHCO₃-Lösung gewaschen. Über Na₂SO₄ wird getrocknet und das Solvens im Vakuum entfernt. Chromatographie an Kieselgel mit EE liefert 32 g eines harzähnlichen Feststoffs.
   R_{f}(EE) = 0.19 MS (FAB): 467 (M+H)⁺
c) 4-{3-[2-(1,3-Dioxo-1 ,3-dihydro-isoindol-2-yl)-ethoxy]-7,12-dihydroxy-10,13-dimethyl-hexadecahydro-cyclopenta[a]phenanthren-17-yl}-pentansäuremethylester
   1.5 g 4-[7,12-Dihydroxy-3-(2-hydroxy-ethoxy)-10,13-dimethyl-hexadecahydrocyclopenta[a]phenanthren-17-yl]-pentansäure-methylester, 950 mg Triphenylphosphin und 550 mg Phthalimid werden in 26 ml THF auf 45 °C erwärmt und bei dieser Temperatur 1,14 ml Azodicarbonsäurediethylester zugetropft. 2 Stunden wird bei 45°C gerührt, anschließend das Reaktionsgemisch in 200 ml einer halbkonzentrierten wäßrigen NaHCO₃-Lösung gegossen und 3 mal mit je 200 ml EE extrahiert. Über Na₂SO₄ wird getrocknet und das Solvens im Vakuum entfernt. Chromatographie an Kieselgel mit *t*-Butylmethylether (MTB) liefert 1.76 g eines zähen Öls.
   R_{f}(EE) = 0.60 MS (FAB) : 602 (M+Li)⁺
d) 4-[3-(2-Amino-ethoxy)-7,12-dihydroxy-10,13-dimethyl-hexadecahydrocyclopenta[a]phenanthren-17-yl]-pentansäure-methylester
   1.7 g 4-{3-[2-(1,3-Dioxo-1,3-dihydro-isoindol-2-yl)-ethoxy]-7,12-dihydroxy-10,13-dimethyl-hexadecahydro-cyclopenta[a]phenanthren-17-yl}-pentansäure-methylester sowie 0.52 ml Hydrazinhydrat (80%) werden in 14 ml Methanol gelöst und 3 Stunden unter Rückfluß gekocht. Anschließend wird auf 40°C abgekühlt und das Reaktionsgemisch mit 8.7 ml einer 2N wäßrigen HCI-Lösung versetzt. 30 Minuten wird bei 40°C nachgerührt, anschließend die flüchtigen Bestandteile im Vakuum entfernt. Chromatographie an Kieselgel mit Aceton/Wasser 10:1 liefert 540 mg harzähnlichen Feststoffs.
   R_{f}(Aceton/Wasser 10:1) = 0.06 MS (FAB): 466 (M+H)⁺
e) 4-[3-(2-Amino-ethoxy)-7,12-dihydroxy-10,13-dimethyl-hexadecahydrocyclopenta[a]phenanthren-17-yl]-pentansäure
   3 g 4-[3-(2-Amino-ethoxy)-7,12-dihydroxy-10,13-dimethyl-hexadecahydrocyclopenta[a]phenanthren-17-yl]-pentansäure-methylester und 310 mg NaOH werden in 5 ml Wasser sowie 30 ml Methanol 24 Stunden bei RT gerührt. Die Solventien werden im Vakuum entfernt, mit 200 ml Wasser aufgenommen und mit wäßriger HCI-Lösung auf pH = 7-7.5 gestellt. 1 Stunde wird nachgerührt und anschließend das Produkt abfiltriert. Man erhält 1.6 g eines blaßgelbe kristallinen Feststoffs. mp 185-195°C.
   R_{f}(CH₂Cl₂/MeOH/Essigsäure/Wasser 32:8:1:1) = 0.18 MS (ES): 452 (M+H)⁺
f) 2-Methyl-3-(3,4,5-trifluoro-phenyl)-acrylsäure-ethylester
   4.3 ml 2-Phosphonopropionsäure-triethylester werden in 30 ml wasserfreiem THF gelöst und bei 0°C 12.5 ml einer 1.6 N Lösung von n-Butyllithium in Hexan zugetropft.15 Minuten wird bei RT gerührt und anschließend eine Lösung von 3.2 g 3,4,5-Trifluorbenzaldehyd in 8 ml wasserfreiem THF zugetropft. Eine Stunde wird bei RT gerührt und 16 Stunden bei RT stehen gelassen. Das Reaktionsgemisch wird mit 300 ml Wasser verdünnt, 30 ml einer gesättigten wäßrigen Na₂CO₃-Lösung zugegeben und 3 mal mit je 100 ml EE extrahiert. Über Na₂SO₄ wird getrocknet und das Solvens im Vakuum entfernt. Chromatographie an Kieselgel mit EE/HEP 1:8 liefert 3.8 g farbloser Kristalle; mp 54°C.
   R_{f}(EE/HEP 1:8) = 0.35 MS (DCI): 245 (M+H)⁺
g) 3-(4-{2-[17-(3-Carboxy-1-methyl-propyl)-7,12-dihydroxy-10,13-dimethylhexadecahydro-cyclopenta[a]phenanthren-3-yloxy]-ethylamino}-3,5-difluorophenyl)-2-methyl-acrylsäure-ethylester
   600 mg 4-[3-(2-Amino-ethoxy)-7,12-dihydroxy-10,13-dimethyl-hexadecahydrocyclopenta[a]phenanthren-17-yl]-pentansäure, 390 mg 2-Methyl-3-(3,4,5-trifluorophenyl)-acrylsäure-ethylester und 828 mg K₂CO₃ werden in 10 ml Dimethylacetamid 2.5 Stunden lang bei 130°C gerührt. Das Reaktionsgemisch wird nach dem Abkühlen mit 400 ml CH₂Cl₂ verdünnt und mit 400 ml einer 5% wäßrigen NaHSO₄-Lösung gewaschen. Über MgSO₄ wird getrocknet und das Solvens im Vakuum entfernt. Chromatographie an Kieselgel mit CH₂Cl₂/MeOH 10:1 liefert 155 mg eines farblosen Öls.
   R_{f}(CH₂Cl₂/MeOH 10:1) = 0.27 MS (ES): 676 (M+H)⁺
i) 4-(3-{2-[2,6-Difluoro-4-(3-guanidino-2-methyl-3-oxo-propenyl)-phenylamino]-ethoxy}-7,12-dihydroxy-10,13-dimethyl-hexadecahydrocyclopenta[a]phenanthren-17-yl)-pentansäure
   130 mg Guanidin-Hydrochlorid und 125 mg Kalium-*t*-Butylat werden in 1 ml wasserfreiem DMF 30 Minuten bei RT gerührt. Anschließend wird eine Lösung von 150 mg 3-(4-{2-[17-(3-Carboxy-1-methyl-propyl)-7,12-dihydroxy-10,13-dimethylhexadecahydro-cyclopenta[a]phenanthren-3-yloxy]-ethylamino}-3,5-difluoro-phenyl)-2-methyl-acrylsäure-ethylester in 1 ml wasserfreiem DMF addiert und 6 Stunden bei 110- 115°C gerührt. Das Reaktionsgemisch wird dann auf 100 ml Wasser gegossen, mit wäßriger HCI-Lösung auf pH = 6 gestellt und das Produkt abfiltriert. Im Feinvakuum wird getrocknet und man erhält 8.0 mg eines amorphen Feststoffs.
   R_{f}(CH₂Cl₂/MeOH/Essigsäure/Wasser 32:8:1:1) = 0.21 MS (ES): 689 (M+H)⁺

## Patentansprüche

1. Verbindungen der Formel I 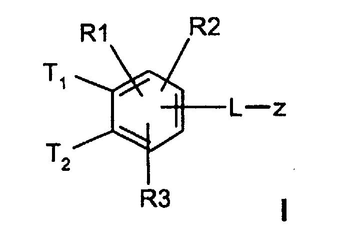 worin bedeuten
T1 und T2 unabhängig voneinander 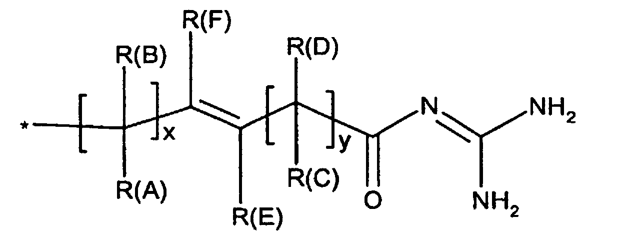 oder Wasserstoff, wobei T1 und T2 nicht gleichzeitig Wasserstoff sein können;
z 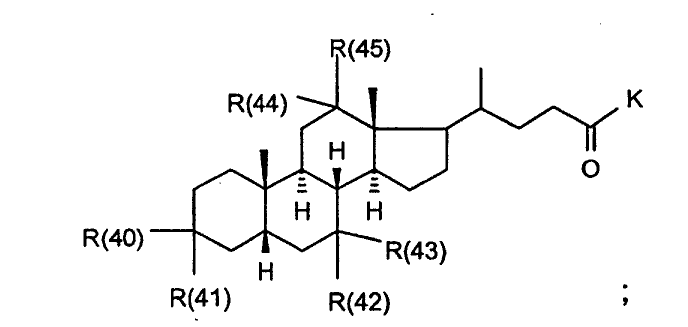
R(A), R(B), R(C), R(D) unabhängig voneinander Wasserstoff, F, Cl, Br, I, CN, OH, NH₂. -(C₁-C₈)-Alkyl, -O-(C₁-C₈)-Alkyl, wobei die Alkylreste ein oder mehrfach mit F substituiert sein können, (C₃-C₈)-Cycloalkyl, Phenyl, Benzyl, NHR(7), NR(7)R(8), O-(C₃-C₆)-Alkenyl, O-(C₃-C₈)-Cycloalkyl, O-Phenyl, O-Benzyl, wobei der Phenylkern bis zu dreifach substituiert sein kann mit F, Cl, CF₃, Methyl, Methoxy, NR(9)R(10);
R(7), R(8) unabhängig voneinander Wasserstoff, -(C₁-C₈)-Alkyl, wobei der Alkylrest ein oder mehrfach mit F substituiert sein kann,
(C₃-C₈)-Cycloalkyl, (C₃-C₆)-Alkenyl, (C₃-C₈)-Cycloalkyl, Phenyl, Benzyl, wobei der Phenylkern bis zu dreifach substituiert sein kann mit F, Cl, CF₃, Methyl, Methoxy, NR(9)R(10); oder
R(7), R(8) bilden gemeinsam eine Kette von 4 oder 5 Methylengruppen, von denen eine CH₂-Gruppe durch Sauerstoff, Schwefel, NH, N-CH₃ oder N-Benzyl ersetzt sein kann;
R(9), R(10) unabhängig voneinander Wasserstoff, (C₁-C₄)-Alkyl, (C₁-C₄)-Perfluoralkyl;
x Null, 1 oder 2;
y Null, 1 oder 2;
R(E), R(F) unabhängig voneinander Wasserstoff, F, Cl, Br, I, CN, (C₁-C₈)-Alkyl, O-(C₁-C₈)-Alkyl, wobei der Alkylrest ein oder mehrfach mit F substituiert sein kann,
(C₃-C₈)-Cycloalkyl, O-(C₃-C₆)-Alkenyl, O-(C₃-C₈)-Cycloalkyl, O-Phenyl, O-Benzyl, wobei der Phenylkern bis zu dreifach substituiert sein kann mit F, Cl. CF₃, Methyl, Methoxy, NR(9)R(10);
R(1), R(2), R(3) unabhängig voneinander Wasserstoff, F, Cl, Br, I, CN, -(C₁-C₈)-Alkyl, -O-(C₁-C₈)-Alkyl, wobei die Alkylreste ein oder mehrfach mit F substituiert sein können,
-(C=O)-N=C(NH₂)₂, -(SO₀₋₂)-(C₁-C₈)-Alkyl, -(SO₂)-NR(7)R(8), -O-(C₀-C₈)-Alkylen-phenyl, -(C₀-C₈)-Alkylen-phenyl, wobei die Phenylkeme bis zu 3-fach substituiert sein können mit F, Cl, CF₃, Methyl, Methoxy, -(C₀-C₈)-Alkylen-NR(9)R(10);
L - O-, -NR(47)-, -(C₁-C₈)-Alkylen-, -(C₂-C₈)-Alkenylen-, -(C₂-C₈)-Alkinylen-, -COO-, -CO-NR(47)-, -SO₂-NR(47)-, -O-(CH₂)ₙ-O-, -NR(47)-(CH₂)ₙ-O-, -NR(48)-CO-(CH₂)ₙ-O-, -CO-NR(48)-(CH₂)ₙ-O-, -O-CO-(CH₂)ₙ-O-, -SO₂-NR(48)-(CH₂)ₙ-O-, -NR(48)-CO-CH₂-CH₂-CO-NR(48)-(CH₂)ₙ-O-, -NR(48)-CO-CH=CH-CO-NR(48)-(CH₂)ₙ-O-, -NR(48)-SO₂-(CH₂)ₙ-O-
R(47) Wasserstoff, (C₁-C₈)-Alkyl, R(48)-CO-, Phenyl, Benzyl;
R(48) Wasserstoff, (C₁-C₈)-Alkyl, Phenyl und Benzyl, wobei der Phenylkem bis zu 3-fach substituiert sein kann mit F, Cl, CF₃,Methyl, Methoxy;
n 1 bis 8;
R(40) bis R(45) unabhängig voneinander Wasserstoff, -OR(50), -SR(50), NHR(50), -NR(50)₂, -O-(CO)-R(50), -S-(CO)-R(50), -NH-(CO)-R(50),-O-PO-(OR(50))-OR(50), -O-(SO₂)-OR(50), -R(50), eine Bindung zu L; oder
R(40) und R(41), R(42) und R(43), R(44) und R(45) bilden jeweils gemeinsam der Sauerstoff einer Carbonylgruppe;
wobei immer genau einer der Reste R(40) bis R(45) die Bedeutung einer Bindung zu L hat;
K -OR(50), -NHR(50), -NR(50)₂, -HN-CH₂-CH₂-CO₂H, -HN-CH₂-CH₂-SO₃H, -NH-CH₂-COOH, -N(CH₃)CH₂CO₂H, -HN-CH(R46)CO₂H, -OKa, wobei Ka ein Kation bedeutet, wie z.B. ein Alkali- oder Erdalkaliion oder ein quartäres Ammoniumion;
R(46) Wasserstoff C₁-C₄-Alkyl, Benzyl, -CH₂-OH, H₃CSCH₂CH₂-, HO₂CCH₂-, HO₂CCH₂CH₂-;
R(50) Wasserstoff, (C₁-C₄)-Alkyl, Phenyl oder Benzyl, wobei der Phenylkern bis zu 3-fach substituiert sein kann mit F, Cl, CF₃,Methyl, Methoxy;
sowie deren pharmazeutisch verträgliche Salze.

2. Verbindungen der Formel I, gemäß Anspruch 1, **dadurch gekennzeichnet, daß** darin bedeuten
T1 und T2 unabhängig voneinander 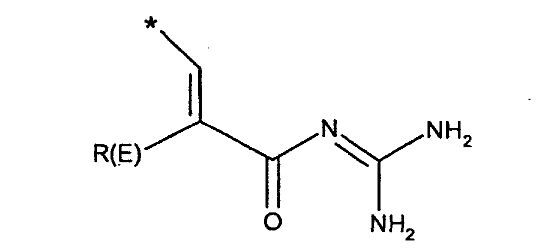 oder Wasserstoff, wobei T1 und T2 nicht gleichzeitig Wasserstoff sein können;
L-z 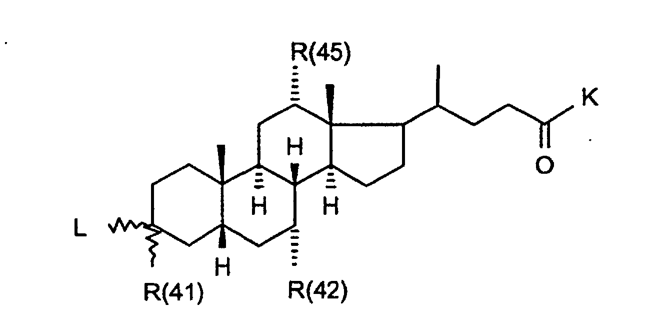
R(E) Wasserstoff, F, Cl, CN, (C₁-C₄)-Alkyl, -O-(C₁-C₄)-Alkyl, wobei die Alkylreste ein oder mehrfach mit F substituiert sein können,
(C₃-C₆)-Cycloalkyl, -O-(C₃-C₈)-Alkenyl, O-(C₃-C₆)-Cycloalkyl, O-Phenyl, O-Benzyl, wobei der Phenylkem bis zu dreifach substituiert sein kann mit F, Cl, CF₃, Methyl, Methoxy, NR(9)R(10);
R(9), R(10) unabhängig voneinander Wasserstoff, CH₃, CF₃;
R(1), R(2), R(3) unabhängig voneinander Wasserstoff, F, Cl, CN, -SO₂-(C₁-C₄)-Alkyl, -SO₂-N((C₁-C₄)-Alkyl)₂, -SO₂-NH(C₁-C₄)-Alkyl, -SO₂-NH₂, -SO₂-(C₁-C₄)-Alkyl, -(C₁-C₄)-Alkyl, -O-(C₁-C₄)-Alkyl, wobei die Alkylreste ein oder mehrfach mit F substituiert sein können,
-O-(C₀-C₄)-Alkylen-phenyl, -(C₀-C₄)-Alkylen-phenyl, wobei die Phenylkeme bis zu 3-fach substituiert sein können mit F, Cl, CF₃, Methyl, Methoxy;
L -O-, -NR(47)-, -(C₁-C₄)-Alkylen-, -(C₂-C₄)-Alkenylen-, -(C₂-C₄)-Alkinylen-, -COO-, -CO-NR(47)-, -SO₂-NR(47)-, -O-(CH₂)ₙ-O-, -NR(47)-(CH₂)ₙ-O-, -NR(48)-CO-(CH₂)ₙ-O-, -CO-NR(48)-(CH₂)ₙ-O-, -SO₂-NR(48)-(CH₂)ₙ-O-;
R(47) Wasserstoff, (C₁-C₄)-Alkyl, R(48)-CO-, Phenyl, Benzyl;
R(48) Wasserstoff, (C₁-C₄)-Alkyl, Phenyl und Benzyl, wobei der Phenylkem bis zu 3-fach substituiert sein kann mit F, Cl, CF₃, Methyl, Methoxy;
n 1-4;
R(41), R(42), R(45) unabhängig voneinander Wasserstoff, -OR(50), NHR(50), -NR(50)₂. -O-(CO)-R(50), -NH-(CO)-R(50);
R(50) Wasserstoff, (C₁-C₄)-Alkyl, Phenyl oder Benzyl,wobei der Phenylkem bis zu 3-fach substituiert sein kann mit F, Cl, CF₃, Methyl, Methoxy;
K -OR(50), -NHR(50), -NR(50)₂, -HN-CH₂-CH₂-CO₂H, -HN-CH₂-CH₂-SO₃H, -NH-CH₂-COOH, -N(CH₃)CH₂CO₂H, -OKa, wobei Ka ein Kation bedeutet, wie z.B. ein Alkali- oder Erdalkaliion oder ein quartäres Ammoniumion;
sowie deren pharmazeutisch verträgliche Salze

3. Verbindungen der Formel I, gemäß Anspruch 1 oder 2, **dadurch gekennzeichnet, daß** darin bedeuten
T1 und T2 unabhängig voneinander 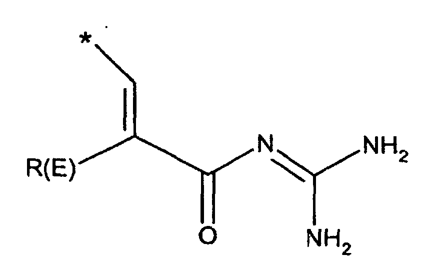 oder Wasserstoff, wobei T1 und T2 nicht gleichzeitig Wasserstoff sein können,
und L-z 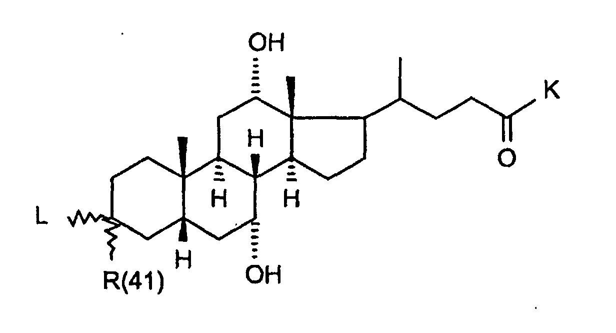
R(E) Wasserstoff, F, Cl, CN, (C₁-C₄)-Alkyl, -O(C₁-C₄)-Alkyl, CF₃ ,-OCF₃;
R(1), R(2) unabhängig voneinander Wasserstoff, F, Cl, CN, -SO₂-CH₃, -(C₁-C₄)-Alkyl, -O-(C₁-C₄)-Alkyl, wobei die Alkylreste ein oder mehrfach mit F substituiert sein können, -O-(C₀-C₄)-Alkylen-phenyl, -(C₀-C₄)-Alkylenphenyl, wobei die Phenylkerne bis zu 3-fach substituiert sein können mit F, Cl, CF₃, Methyl, Methoxy;
R(3) Wasserstoff,
L -O-, -NR(47)-, -CH₂-CH₂-, CH=CH-, -(C≡C)-, -COO-, -CO-NR(47)-, -SO₂-NR(47)-, -O-(CH₂)ₙ-O-, -NR(47)-(CH₂)ₙ-O-, -NR(48)-CO-(CH₂)ₙ-O-, -CO-NR(48)-(CH₂)ₙ-O-, -SO₂-NR(48)-(CH₂)ₙ-O-;
R(47) Wasserstoff, (C₁-C₄)-Alkyl, R(48)-CO-, Phenyl, Benzyl;
R(48) Wasserstoff, (C₁-C₄)-Alkyl, Phenyl und Benzyl, wobei der Phenylkern bis zu 3-fach substituiert sein kann mit F, Cl, CF₃, Methyl, Methoxy;
n 1-4;
R(41) Wasserstoff, -OH;
K -OR(50), -NHR(50), -NR(50)₂, -HN-CH₂-CH₂-CO₂H, -HN-CH₂-CH₂-SO₃H, -NH-CH₂-COOH, -N(CH₃)CH₂CO₂H, -OKa, wobei Ka ein Kation bedeutet, wie z.B. ein Alkali- oder Erdalkaliion oder ein quartäres Ammoniumion;
R(50) Wasserstoff, (C₁-C₄)-Alkyl, Phenyl oder Benzyl,wobei der Phenylkern bis zu 3-fach substituiert sein kann mit F, Cl, CF₃, Methyl, Methoxy;
sowie deren pharmazeutisch verträgliche Salze.

4. Verbindungen der Formel I, gemäß einem oder mehreren der Ansprüche 1 bis 3, **dadurch gekennzeichnet, daß** Formel I die Struktur la aufweist 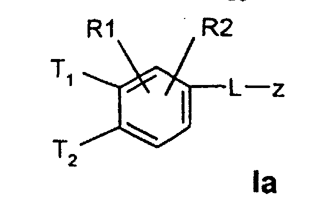 worin bedeuten
T1 und T2 unabhängig voneinander 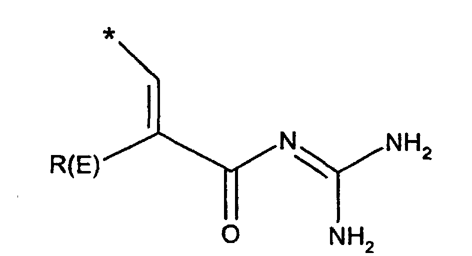 oder Wasserstoff, wobei T1 und T2 nicht gleichzeitig Wasserstoff sein können;
L-z 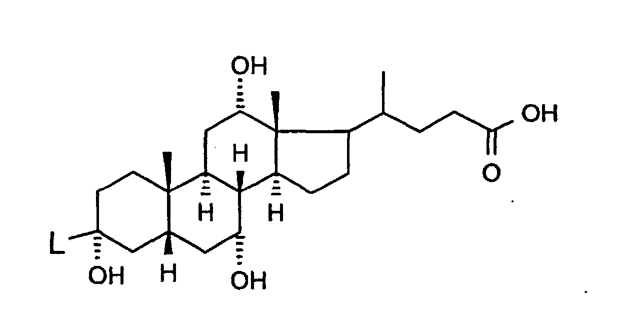
L ―C≡C― , -NH-CH₂-CH₂-O-;
R(E) Wasserstoff, (C₁-C₄)-Alkyl;
R(1), R(2) unabhängig voneinander Wasserstoff, F, Cl, CN, -SO₂-CH₃, -(C₁-C₄)-Alkyl, -O-(C₁-C₄)-Alkyl, wobei die Alkylreste ein oder mehrfach mit F substituiert sein können;
sowie deren pharmazeutisch verträgliche Salze.

5. Arzneimittel enthaltend eine oder mehrere der Verbindungen gemäß einem oder mehreren der Ansprüche 1 bis 4.

6. Arzneimittel gemäß Anspruch 5 enthaltend ein oder mehrere lipidsenkende Wirkstoffe.

7. Verbindungen gemäß einem oder mehreren der Ansprüche 1 bis 4 zur Anwendung als Medikament zur Prophylaxe oder Behandlung von Gallensteinen.

8. Verfahren zur Herstellung eines Arzneimittels enthaltend eine oder mehrere der Verbindungen gemäß einem oder mehreren der Ansprüche 1 bis 4, **dadurch gekennzeichnet, daß** der Wirkstoff mit einem pharmazeutisch geeigneten Träger vermischt wird und diese Mischung in eine für die Verabreichung geeignete Form gebracht wird.

9. Verwendung der Verbindungen gemäß einem oder mehreren der Ansprüche 1 bis 4 zur Herstellung eines Medikaments zur Prophylaxe oder Behandlung von Gallensteinen.

## Claims

1. A compound of the formula I 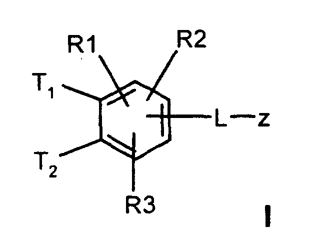 in which
T1 and T2 independently of one another are 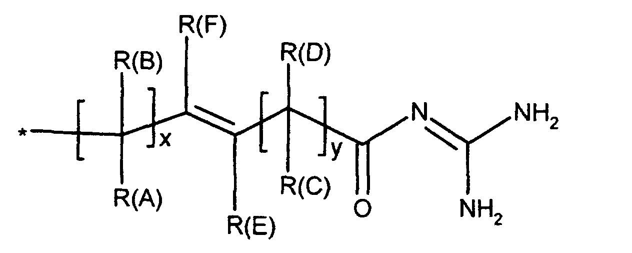 or hydrogen, where T1 and T2 cannot simultaneously be hydrogen;
z is 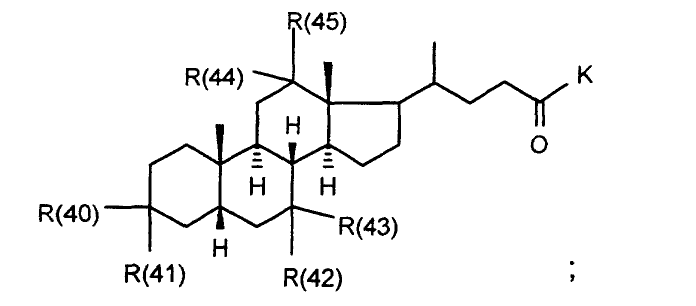
R(A), R(B), R(C), R(D) independently of one another are hydrogen, F, Cl, Br, I, CN, OH, NH₂, -(C₁-C₈)-alkyl, -O-(C₁-C₈)-alkyl, where the alkyl radicals can be substituted one or more times by F; (C₃-C₈)-cycloalkyl, phenyl, benzyl, NHR(7), NR(7)R(8), O-(C₃-C₆)-alkenyl, O-(C₃-C₈)-cycloalkyl, O-phenyl, O-benzyl, where the phenyl nucleus can be substituted up to three times by F, Cl, CF₃, methyl, methoxy, NR(9)R(10);
R(7), R(8) independently of one another are hydrogen, -(C₁-C₈)-alkyl, where the alkyl radical can be substituted one or more times by F,
(C₃-C₈)-cycloalkyl, (C₃-C₆)-alkenyl, (C₃-C₈)-cycloalkyl, phenyl, benzyl, where the phenyl nucleus can be substituted up to three times by F, Cl, CF₃, methyl, methoxy, NR(9)R(10); or
R(7), R(8) together form a chain of 4 or 5 methylene groups, of which one CH₂ group can be replaced by oxygen, sulfur, NH, N-CH₃ or N-benzyl;
R(9), R(10) independently of one another are hydrogen, (C₁-C₄)-alkyl, (C₁-C₄)-perfluoroalkyl;
x is zero, 1 or 2;
y is zero, 1 or 2;
R(E), R(F) independently of one another are hydrogen, F, Cl, Br, I, CN, (C₁-C₈)-alkyl, O-(C₁-C₈)-alkyl, where the alkyl radical can be substituted one or more times by F,
(C₃-C₈)-cycloalkyl, 0- (C₃-C₆)-alkenyl, O-(C₃-C₈)-cycloalkyl, O-phenyl, O-benzyl, where the phenyl nucleus can be substituted up to three times by F, Cl, CF₃, methyl, methoxy, NR(9)R(10);
R(1), R(2), R(3) independently of one another are hydrogen, F, Cl, Br, I, CN,-(C₁-C₈)-alkyl, -O-(C₁-C₈)-alkyl, where the alkyl radicals can be substituted one or more times by F, - (C=O)-N=C(NH₂)₂, - (SO₀₋₂)-(C₁-C₈)-alkyl, -(SO₂)-NR(7)R(8), -O-(C₀-C₈)-alkylenephenyl, - (C₀-C₈)-alkylenephenyl, where the phenyl nuclei can be substituted up to 3 times by F, Cl, CF₃, methyl, methoxy, -(C₀-C₈)-alkylene-NR(9)R(10);
L is -O-, -NR(47)-, -(C₁-C₈)-alkylene-, - (C₂-C₈)-alkenylene-, - (C₂-C₈)-alkynylene-, -COO-, -CO-NR(47)-, -SO₂-NR(47)-, -O-(CH₂)ₙ-O-, -NR(47)-(CH₂)ₙ-O-, -NR(48)-CO-(CH₂)ₙ-O-, -CO-NR(48)-(CH₂)ₙ-O-, -O-CO-(CH₂)ₙ-O-, -SO₂-NR(48)-(CH₂)ₙ-O-, -NR(48)-CO-CH₂-CH₂-CO-NR(48)-(CH₂)ₙ-O-, -NR(48)-CO-CH=CH-CO-NR(48)-(CH₂)ₙ-O-, -NR(48)-SO₂-(CH₂)ₙ-O-;
R(47) is hydrogen, (C₁-C₈)-alkyl, R(48)-CO-, phenyl, benzyl;
R(48) is hydrogen, (C₁-C₈)-alkyl, phenyl and benzyl, where the phenyl nucleus can be substituted up to 3 times by F, Cl, CF₃, methyl, methoxy;
n is 1 to 8;
R(40) to R(45) independently of one another are hydrogen, -OR(50), -SR(50), NHR(50), -NR(50)₂, -O-(CO)-R(50), -S-(CO)-R(50), -NH-(CO)-R(50), -O-PO-(OR(50))-OR(50), -O-(SO₂)-OR(50), -R(50), a bond to L; or
R(40) and R(41), R(42) and R(43), R(44) and R(45) in each case together form the oxygen of a carbonyl group;
where always just one of the radicals R(40) to R(45) has the meaning of a bond to L;
K is -OR(50), -NHR(50), -NR(50)₂, -HN-CH₂-CH₂-CO₂H, -HN-CH₂-CH₂-SO₃H, -NH-CH₂-COOH, -N(CH₃)CH₂CO₂H, -HN-CH(R46)CO₂H, -OKa, where Ka is a cation, such as, for example, an alkali metal or alkaline earth metal ion or a quaternary ammonium ion;
R(46) is hydrogen, C₁-C₄-alkyl, benzyl, -CH₂-OH, H₃CSCH₂CH₂-, HO₂CCH₂-, HO₂CCH₂CH₂-;
R(50) is hydrogen, (C₁-C₄)-alkyl, phenyl or benzyl, where the phenyl nucleus can be substituted up to 3 times by F, Cl, CF₃, methyl, methoxy;
or its pharmaceutically tolerable salts.

2. A compound of the formula I, as claimed in claim 1, wherein
T1 and T2 independently of one another are 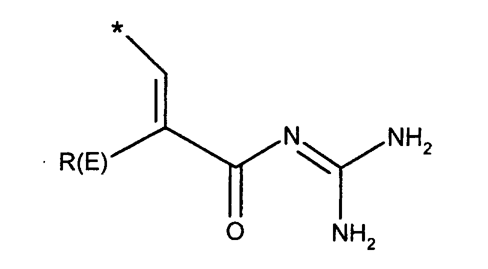 or hydrogen, where T1 and T2 cannot simultaneously be hydrogen;
L-z is 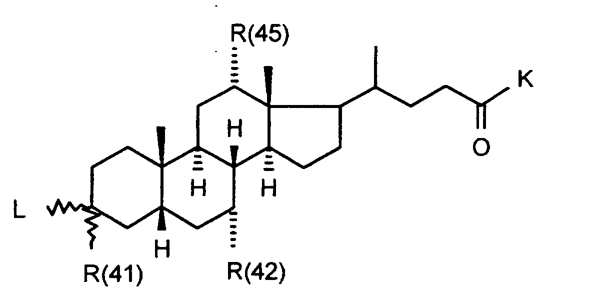
R(E) is hydrogen, F, Cl, CN, (C₁-C₄)-alkyl, -O-(C₁-C₄)-alkyl, where the alkyl radicals can be substituted one or more times by F, (C₃-C₆)-cycloalkyl, -O-(C₃-C₈)-alkenyl, O-(C₃-C₆)-cycloalkyl, O-phenyl, O-benzyl, where the phenyl nucleus can be substituted up to three times by F, Cl, CF₃, methyl, methoxy, NR(9)R(10);
R(9), R(10) independently of one another are hydrogen, CH₃, CF₃;
R(1), R(2), R(3) independently of one another are hydrogen, F, Cl, CN, -SO₂-(C₁-C₄)-alkyl, -SO₂-N((C₁-C₄)-alkyl)₂, -SO₂-NH(C₁-C₄)-alkyl, -SO₂-NH₂, -SO₂-(C₁-C₄)-alkyl, -(C₁-C₄)-alkyl, -O-(C₁-C₄)-alkyl, where the alkyl radicals can be substituted one or more times by F,
-O-(C₀-C₄)-alkylenephenyl, -(C₀-C₄)-alkylenephenyl, where the phenyl nuclei can be substituted up to 3 times by F, Cl, CF₃, methyl, methoxy;
L is -O-, -NR(47)-, -(C₁-C₄)-alkylene-, - (C₂-C₄)-alkenylene-, -(C₂-C₄)-alkynylene-, -COO-, -CO-NR(47)-, -SO₂-NR(47)-, -O-(CH₂)n-O-, -NR(47)-(CH₂)ₙ-O-, -NR(48)-CO-(CH₂)ₙ-O-, -CO-NR(48)-(CH₂)ₙ-O-, -SO₂-NR(48)-(CH₂)ₙ-O-;
R(47) is hydrogen, (C₁-C₄)-alkyl, R(48)-CO-, phenyl, benzyl;
R(48) is hydrogen, (C₁-C₄)-alkyl, phenyl and benzyl, where the phenyl nucleus can be substituted up to 3 times by F, Cl, CF₃, methyl, methoxy;
n is 1-4;
R(41), R(42), R(45) independently of one another are hydrogen, -OR(50), NHR(50), -NR(50)₂, -O-(CO)-R(50), -NH-(CO)-R(50);
R(50) is hydrogen, (C₁-C₄)-alkyl, phenyl or benzyl, where the phenyl nucleus can be substituted up to 3 times by F, Cl, CF₃, methyl, methoxy;
K is -OR(50), -NHR(50), -NR(50)₂, -HN-CH₂-CH₂-CO₂H, -HN-CH₂-CH₂-SO₃H, -NH-CH₂-COOH, -N(CH₃)CH₂CO₂H, -OKa, where Ka is a cation, such as, for example, an alkali metal or alkaline earth metal ion or a quaternary ammonium ion;
or its pharmaceutically tolerable salts.

3. A compound of the formula I, as claimed in claim 1 or 2, wherein
T1 and T2 independently of one another are 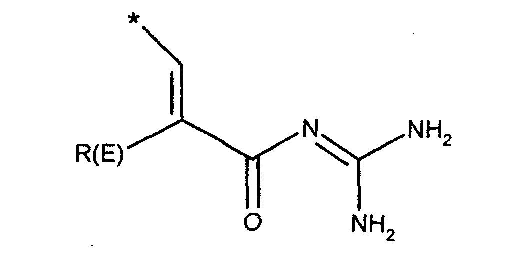 or hydrogen, where T1 and T2 cannot simultaneously be hydrogen,
and L-z is 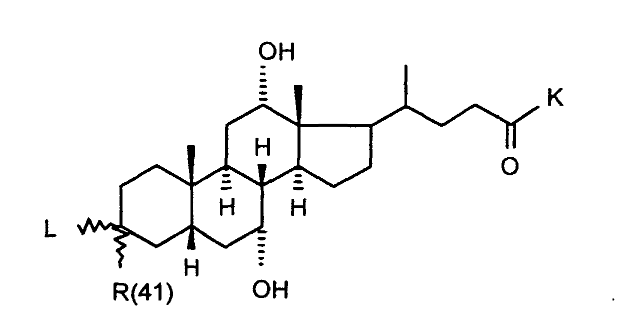
R(E) is hydrogen, F, Cl, CN, (C₁-C₄)-alkyl, -O(C₁-C₄)-alkyl, CF₃, -OCF₃;
R(1), R(2) independently of one another are hydrogen, F, Cl, CN, -SO₂-CH₃, -(C₁-C₄)-alkyl, -O-(C₁-C₄)-alkyl, where the alkyl radicals can be substituted one or more times by F; -O-(C₀-C₄)-alkylenephenyl, - (C₀-C₄)-alkylenephenyl, where the phenyl nuclei can be substituted up to 3 times by F, Cl, CF₃, methyl, methoxy;
R(3) is hydrogen;
L is -O-, -NR(47)-, -CH₂-CH₂-, CH=CH-, -(C≡C)-, -COO-, -CO-NR(47)-, -SO₂-NR(47)-, -O-(CH₂)ₙ-O-, -NR(47)-(CH₂)ₙ-O-, -NR(48)-CO-(CH₂)ₙ-O-, -CO-NR(48)-(CH₂)ₙ-O-, -SO₂-NR(48)-(CH₂)ₙ-O-;
R(47) is hydrogen, (C₁-C₄)-alkyl, R(48)-CO-, phenyl, benzyl;
R(48) is hydrogen, (C₁-C₄)-alkyl, phenyl and benzyl, where the phenyl nucleus can be substituted up to 3 times by F, Cl, CF₃, methyl, methoxy;
n is 1-4;
R(41) is hydrogen, -OH;
K is -OR(50), -NHR(50), -NR(50)₂, -HN-CH₂-CH₂-CO₂H, -HN-CH₂-CH₂-SO₃H, -NH-CH₂-COOH, -N(CH₃)CH₂CO₂H, -OKa, where Ka is a cation, such as, for example, an alkali metal or alkaline earth metal ion or a quaternary ammonium ion;
R(50) is hydrogen, (C₁-C₄)-alkyl, phenyl or benzyl, where the phenyl nucleus can be substituted up to 3 times by F, Cl, CF₃, methyl, methoxy;
or its pharmaceutically tolerable salts.

4. A compound of the formula I, as claimed in one or more of claims 1 to 3, wherein formula I has the structure Ia 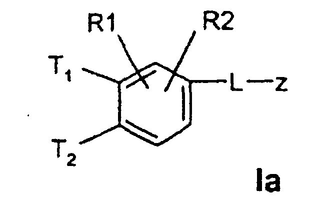 in which
T1 and T2 independently of one another are 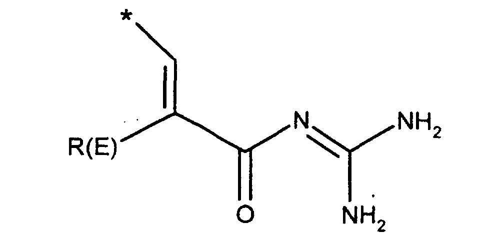 or hydrogen, where T1 and T2 cannot simultaneously be hydrogen;
L-z is 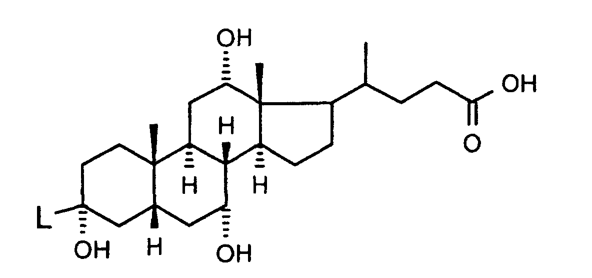
L is ―C≡C―, -NH-CH₂-Ch₂-O-;
R(E) is hydrogen, (C₁-C₄)-alkyl;
R(1), R(2) independently of one another are hydrogen, F, Cl, CN, -SO₂-CH₃, -(C₁-C₄)-alkyl, -O-(C₁-C₄)-alkyl, where the alkyl radicals can be substituted one or more times by F;
or its pharmaceutically tolerable salts.

5. A pharmaceutical comprising one or more of the compounds as claimed in one or more of claims 1 to 4.

6. A pharmaceutical as claimed in claim 5 comprising one or more lipid-lowering active compounds.

7. A compound as claimed in one or more of claims 1 to 4 for use as a medicament for the prophylaxis or treatment of gallstones.

8. A process for the production of a pharmaceutical comprising one or more of the compounds as claimed in one or more of claims 1 to 4, which comprises mixing the active compound with a pharmaceutically suitable excipient and bringing this mixture into a form suitable for administration.

9. The use of the compounds as claimed in one or more of claims 1 to 4 for the production of a medicament for the prophylaxis or treatment of gallstones.

## Revendications

1. Composés de formule I 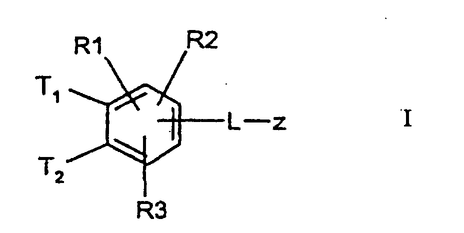 dans laquelle
T1 et T2 représentent, indépendamment l'un de l'autre, un reste 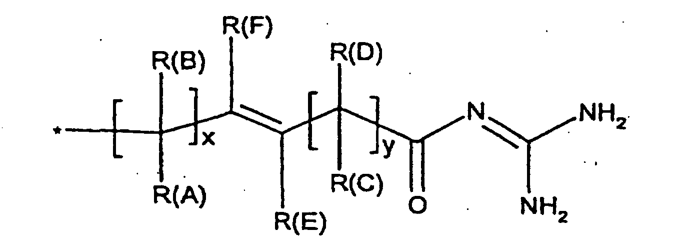 ou un atome d'hydrogène, T1 et T2 ne pouvant être en même temps des atomes d'hydrogène;
z représente un reste 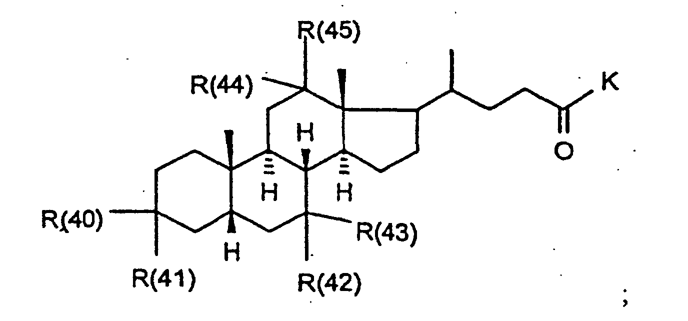
R(A), R(B), R(C), R(D) représentent, indépendamment les uns des autres, un atome d'hydrogène ou un reste F, Cl, Br, I, CN, OH, NH₂, alkyle en C₁-C₈, -O-alkyle en C₁-C₈, les restes alkyle pouvant être substitués par F une ou plusieurs fois, cycloalkyle en C₃-C₈, phényle, benzyle, NHR(7), NR(7)R(8), -O-alcényle en C₃-C₆, -O-cycloalkyle en C₃-C₈, -O-phényle, -O-benzyle, le noyau phényle pouvant être substitue jusqu'à trois fois par F, Cl, CF₃, méthyle, méthoxy, NR(9)R(10);
R(7) et R(8) représentent, indépendamment l'un de l'autre, un atome d'hydrogène, un reste alkyle en C₁-C₈, le reste alkyle pouvant être substitué par F une ou plusieurs fois, cycloalkyle en C₃-C₈, alcényle en C₃-C₆, cycloalkyle en C₃-C₈, phényle, benzyle, le cycle phényle pouvant être substitué jusqu'à trois fois par F, Cl, CF₃, méthyle, méthoxy, NR(9)R(10); ou
R(7) et R(8) forment ensemble une chaîne de 4 ou 5 groupes méthylène, dont un groupe CH₂ peut être remplacé par de l'oxygène, du soufre, NH, N-CH₃ ou N-benzyle;
R(9) et R(10) représentent, indépendamment l'un de l'autre, un atome d'hydrogène ou un reste alkyle en C₁-C₄ ou perfluoroalkyle en C₁-C₄;
x est 0, 1 ou 2;
y est 0, 1 ou 2;
R(E) et R(F) représentent, indépendamment l'un de l'autre, un atome d'hydrogène ou un reste F, Cl, Br, I, CN, alkyle en C₁-C₈, -O-alkyle en C₁-C₈, le reste alkyle pouvant être substitué par F une ou plusieurs fois, cycloalkyle en C₃-C₈, -O-alcényle en C₃-C₆, -O-cycloalkyle en C₃-C₈, -O-phényle, -O-benzyle, le noyau phényle pouvant être substitué jusqu'à trois fois par F, Cl, CF₃, méthyle, méthoxy, NR(9)R(10);
R(1), R(2) et R(3) représentent, indépendamment les uns des autres, un atome d'hydrogène ou un reste F, Cl, Br, I, CN, alkyle en C₁-C₈, -O-alkyle en C₁-C₈, les restes alkyle pouvant être substitués par F une ou plusieurs fois, -(C=O)-N=C(NH₂)₂, -(SO₀₋₂)-alkyle en C₁-C₈, -(SO₂)-NR(7)R(8),
-O-(alkylène en C₀-C₈)-phényle, (alkylène en C₀-C₈)-phényle, les noyaux phényle pouvant être substitués jusqu'à 3 fois par F, Cl, CF₃, méthyle, méthoxy, (alkylène en C₀-C₈)-NR(9)R(10);
L représente un reste -O-, -NR(47)-, alkylène en C₁-C₈, alcénylène en C₂-C₈, alcynylène en C₂-C₈, -COO-, -CO-NR(47)-, -SO₂-NR(47)-, -O-(CH₂)ₙ-O-, -NR(47)-(CH₂)ₙ-O-, -NR(48)-CO-(CH₂)ₙ-O-, -CO-NR(48)-(CH₂)ₙ-O-, -O-CO-(CH₂)ₙ-O-, -SO₂-NR(48)-(CH₂)ₙ-O-, -NR(48)-CO-CH₂-CH₂-CO-NR(48)-(CH₂)ₙ-O-, -NR(48)-CO-CH=CH-CO-NR(48)-(CH₂)ₙ-O-, -NR(48)-SO₂-(CH₂)ₙ-O-;
R(47) représente un atome d'hydrogène ou un reste alkyle en C₁-C₈, R(48)-CO-, phényle, benzyle;
R(48) représente un atome d'hydrogène ou un reste alkyle en C₁-C₈, phényle ou benzyle, le cycle phényle pouvant être substitué jusqu'à 3 fois par F, Cl, CF₃, méthyle, méthoxy;
n est un nombre de 1 à 8;
R(40) à R(45) représentent, indépendamment les uns des autres, un atome d'hydrogène ou un reste -OR(50), -SR(50), -NHR(50), -NR(50)₂, -O-(CO)-R(50), -S-(CO)-R(50), -NH-(CO)-R(50), -O-PO-(OR(50))-OR(50), -O-(SO₂)-OR(50), -R(50), une liaison à L; ou
R(40) et R(41), R(42) et R(43), R(44) et R(45) forment ensemble l'atome d'oxygène d'un groupe carbonyle;
à condition que juste l'un des restes R(40) à R(45) représente une liaison à L;
K représente un reste -OR(50), -NHR(50), -NR(50)₂, -HN-CH₂-CH₂-CO₂H, -HN-CH₂-CH₂-SO₃H, -NH-CH₂-COOH, -N(CH₃)CH₂CO₂H,
-HN-CH(R46)CO₂H, -OKa où Ka représente un cation comme, par exemple, un ion de métal alcalin ou de métal alcalino-terreux ou un ion d'ammonium quaternaire;
R(46) représente un atome d'hydrogène ou un reste alkyle en C₁-C₄, benzyle, -CH₂-OH, H₃CSCH₂CH₂-, HO₂CCH₂-, HO₂CCH₂CH₂-;
R(50) représente un atome d'hydrogène ou un reste alkyle en C₁-C₄, phényle ou benzyle, le noyau phényle pouvant être substitué jusqu'à 3 fois par F, Cl, CF₃, méthyle, méthoxy;
et leurs sels pharmaceutiquement acceptables.

2. Composés de formule I selon la revendication 1, **caractérisés en ce que**
T1 et T2 représentent, indépendamment l'un de l'autre, un reste 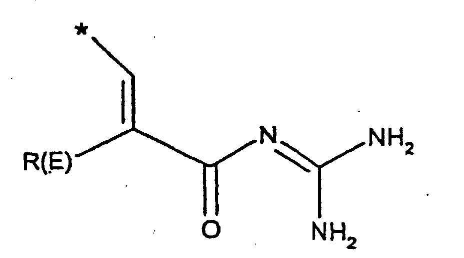 ou un atome d'hydrogène, T1 et T2 ne pouvant être en même temps des atomes d'hydrogène;
L-z représente un reste 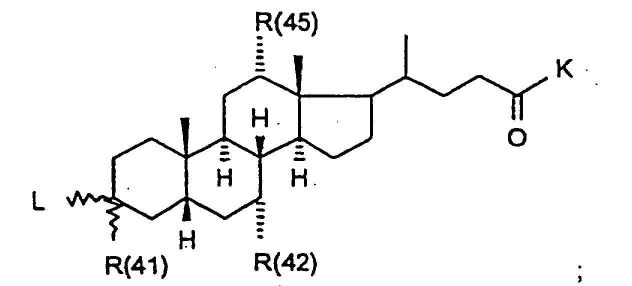
R(E) représente un atome d'hydrogène ou un reste F, Cl, CN, alkyle en C₁-C₄, -O-alkyle en C₁-C₄, les restes alkyle pouvant être substitués par F une ou plusieurs fois, cycloalkyle en C₃-C₆, -O-alcényle en C₃-C₈, -O-cycloalkyle en C₃-C₆, -O-phényle, -O-benzyle, le noyau phényle pouvant être substitué jusqu'à trois fois par F, Cl, CF₃, méthyle, méthoxy, NR(9)R(10);
R(9) et R(10) représentent, indépendamment l'un de l'autre, un atome d'hydrogène ou un reste CH₃ ou CF₃;
R(1), R(2) et R(3) représentent, indépendamment les uns des autres, un atome d'hydrogène ou un reste F, Cl, CN, -(SO₂)-alkyle en C₁-C₄, -(SO₂)-N(alkyle en C₁-C₄)₂, -SO₂-NH(alkyle en C₁-C₄), -SO₂-NH₂, -SO₂-(alkyle en C₁-C₄), alkyle en C₁-C₄, -O-alkyle en C₁-C₄, les restes alkyle pouvant être substitués par F une ou plusieurs fois, -O-(alkylène en C₀-C₄)-phényle, (alkylène en C₀-C₄)-phényle, les noyaux phényle pouvant être substitués jusqu'à 3 fois par F, Cl, CF₃, méthyle, méthoxy;
L représente un reste -O-, -NR(47)-, alkylène en C₁-C₄, alcénylène en C₂-C₄, alcynylène en C₂-C₄, -COO-, -CO-NR(47)-, -SO₂-NR(47)-, -O-(CH₂)ₙ-O-, -NR(47)-(CH₂)ₙ-O-, -NR(48)-CO-(CH₂)ₙ-O-, -CO-NR(48)-(CH₂)ₙ-O-, -SO₂-NR(48)-(CH₂)ₙ-O-;
R(47) représente un atome d'hydrogène ou un reste alkyle en C₁-C₄, R(48)-CO-, phényle, benzyle;
R(48) représente un atome d'hydrogène ou un reste alkyle en C₁-C₄, phényle ou benzyle, le cycle phényle pouvant être substitué jusqu'à 3 fois par F, Cl, CF₃, méthyle, méthoxy;
n est un nombre de 1 à 4;
R(41), R(42) et R(45) représentent, indépendamment les uns des autres, un atome d'hydrogène ou un reste -OR(50), -NHR(50), -NR(50)₂, -O-(CO)-R(50), -NH-(CO)-R(50);
R(50) représente un atome d'hydrogène ou un reste alkyle en C₁-C₄, phényle ou benzyle, le noyau phényle pouvant être substitué jusqu'à 3 fois par F, Cl, CF₃, méthyle, méthoxy;
K représente un reste -OR(50), -NHR(50), -NR(50)₂, -HN-CH₂-CH₂-CO₂H, -HN-CH₂-CH₂-SO₃H, -NH-CH₂-COOH, -N(CH₃)CH₂CO₂H, -OKa où Ka représente un cation comme, par exemple, un ion de métal alcalin ou de métal alcalino-terreux ou un ion d'ammonium quaternaire;
et leurs sels pharmaceutiquement acceptables.

3. Composés de formule I selon la revendication 1 ou 2, **caractérisés en ce que**
T1 et T2 représentent, indépendamment l'un de l'autre, un reste 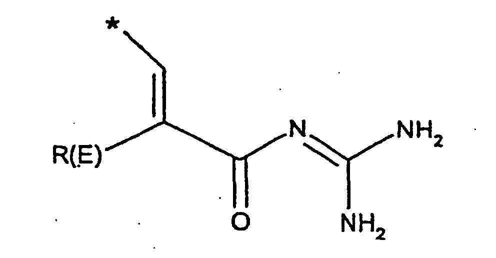 ou un atome d'hydrogène, T1 et T2 ne pouvant être en même temps des atomes d'hydrogène; et
L-z représente un reste 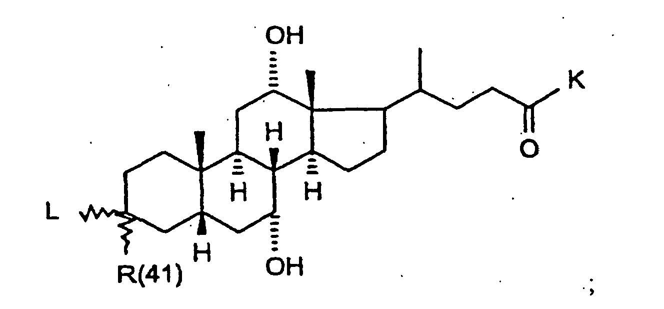
R(E) représente un atome d'hydrogène ou un reste F, Cl, CN, alkyle en C₁-C₄, -O-alkyle en C₁-C₄, CF₃, -OCF₃;
R(1), R(2) représentent, indépendamment l'un de l'autre, un atome d'hydrogène ou un reste F, Cl, CN, -SO₂-CH₃, alkyle en C₁-C₄, -O-alkyle en C₁-C₄, les restes alkyle pouvant être substitués par F une ou plusieurs fois, -O-(alkylène en C₀-C₄)-phényle, (alkylène en C₀-C₄)-phényle, les noyaux phényle pouvant être substitués jusqu'à 3 fois par F, Cl, CF₃, méthyle, méthoxy;
R(3) représente un atome d'hydrogène;
L représente un reste -O-, -NR(47)-, -CH₂-CH₂-, -CH=CH-, -(C≡C)-, -COO-, -CO-NR(47)-, -SO₂-NR(47)-, -O-(CH₂)ₙ-O-, -NR(47)-(CH₂)ₙ-O-, -NR(48)-CO-(CH₂)ₙ-O-, -CO-NR(48)-(CH₂)ₙ-O-, -SO₂-NR(48)-(CH₂)ₙ-O-,
R(47) représente un atome d'hydrogène ou un reste alkyle en C₁-C₄, R(48)-CO-, phényle, benzyle;
R(48) représente un atome d'hydrogène ou un reste alkyle en C₁-C₄, phényle ou benzyle, le cycle phényle pouvant être substitué jusqu'à 3 fois par F, Cl, CF₃, méthyle, méthoxy;
n est un nombre de 1 à 4;
R(41) représente un atome d'hydrogène ou un reste -OH;
K représente un reste -OR(50), -NHR(50), -NR(50)₂, -HN-CH₂-CH₂-CO₂H, -HN-CH₂-CH₂-SO₃H, -NH-CH₂-COOH, -N(CH₃)CH₂CO₂H, -OKa où Ka représente un cation comme, par exemple, un ion de métal alcalin ou de métal alcalino-terreux ou un ion d'ammonium quaternaire;
R(50) représente un atome d'hydrogène ou un reste alkyle en C₁-C₄, phényle ou benzyle, le cycle phényle pouvant être substitué jusqu'à 3 fois par F, Cl, CF₃, méthyle, méthoxy;
et leurs sels pharmaceutiquement acceptables.

4. Composés de formule I selon l'une ou plusieurs des revendications 1 à 3, **caractérisés en ce que** la formule I présente la structure Ia 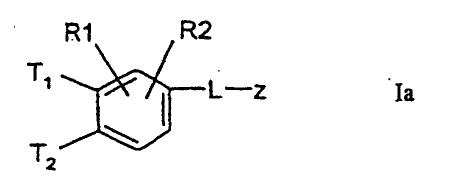 dans laquelle
T1 et T2 représentent, indépendamment l'un de l'autre, un reste 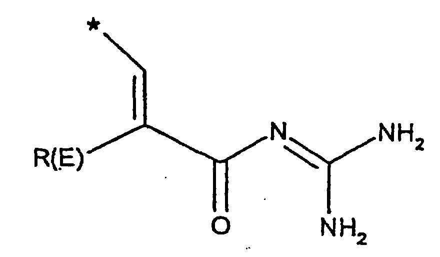 ou un atome d'hydrogène, T1 et T2 ne pouvant être en même temps des atomes d'hydrogène; et
L-z représente un reste 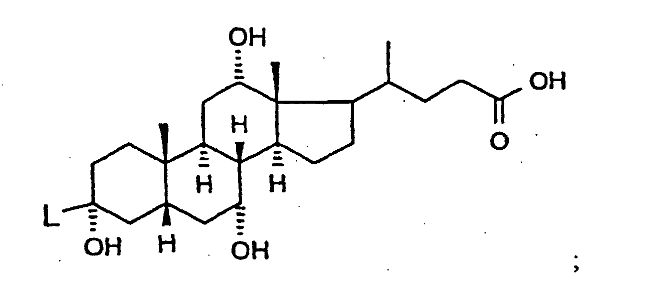
L représente un reste -C≡C-, -NH-CH₂-CH₂-O-;
R(E) représente un atome d'hydrogène ou un reste alkyle en C₁-C₄;
R(1), R(2) représentent, indépendamment l'un de l'autre, un atome d'hydrogène ou un reste F, Cl, CN, -SO₂-CH₃, alkyle en C₁-C₄, -O-alkyle en C₁-C₄, les restes alkyle pouvant être substitués une ou plusieurs fois par F;
et leurs sels pharmaceutiquement acceptables.

5. Médicaments contenant un ou plusieurs des composés selon l'une ou plusieurs des revendications 1 à 4.

6. Médicaments selon la revendication 5, contenant une ou plusieurs substances hypolipémiantes.

7. Composés selon l'une ou plusieurs des revendications 1 à 4 à utiliser comme médicaments pour la prophylaxie ou le traitement de calculs biliaires.

8. Procédé de préparation d'un médicament contenant un ou plusieurs des composés selon l'une ou plusieurs des revendications 1 à 4, **caractérisé en ce que** l'on mélange le composé actif avec un support pharmaceutiquement approprié et on met ce mélange sous une forme appropriée à l'administration.

9. Utilisation des composés selon l'une ou plusieurs des revendications 1 à 4 pour la préparation d'un médicament destiné à la prophylaxie ou au traitement de calculs biliaires.
